(19) Europäisches Patentamt
European Patent Office
Office européen des brevets

(11) **EP 2 815 767 A1**

(12) **EUROPEAN PATENT APPLICATION**

(43) Date of publication:
**24.12.2014 Bulletin 2014/52**

(51) Int Cl.:
***A61K 39/395*** (2006.01)   ***C07K 16/18*** (2006.01)

(21) Application number: **14177646.8**

(22) Date of filing: **16.05.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **14.05.2004 US 571444 P**

(62) Document number(s) of the earlier application(s) in accordance with Art. 76 EPC:
**05779924.9 / 1 755 674**

(71) Applicant: **Alexion Pharmaceuticals, Inc.
Cheshire, CT 06410 (US)**

(72) Inventors:
• **Rother, Russell P.**
**Oklahoma City, OK 73151 (US)**

• **Wang, Hao**
**London, Ontario N5X 4H2 (CA)**
• **Zhong, Zhen**
**deceased (CA)**

(74) Representative: **Vossius & Partner
Siebertstrasse 4
81675 München (DE)**

Remarks:
This application was filed on 18-07-2014 as a divisional application to the application mentioned under INID code 62.

(54) **Prolongation of survival of an allograft by inhibiting complement activity**

(57)    Methods of prolonging survival of allotransplanted cells, tissues or organs are presented. These methods are directed to administering to the allotransplanted recipient an inhibitor of complement activity together with one or more immunosuppressants. The inhibitor of complement activity is administered chronically. These methods have been determined to aid in preventing chronic rejection of allografts. These methods can additionally be used in cases in which the recipient has been presensitized to the allograft or in which there is an ABO mismatch between the allograft and the recipient.

Figure 2A

**Description**

RELATED APPLICATIONS

This application claims the benefit of and priority to U.S. Provisional Patent Application No. 60/571,444, filed May 14, 2004, the disclosure of which is incorporated herein by reference in its entirety.

TECHNICAL FIELD

The present disclosure relates to methods for prolonging survival of an allograft in a mammal. In particular, the present disclosure relates to prolonging survival of an allograft by administering an inhibitor of complement or terminal complement formation, especially an inhibitor of complement C5 cleavage, in addition to one or more drugs that are immunosuppressant.

BACKGROUND

Organ transplantation is the preferred treatment for most patients with chronic organ failure. Although transplantation of kidney, liver, lung, and heart offers an excellent opportunity for rehabilitation as recipients return to a more normal lifestyle, it is limited by the medical/surgical suitability of potential recipients, an increasing shortage of donors, and premature failure of transplanted organ function.

Transplantation of cells, tissues and organs has become very common and is often a life-saving procedure. Organ transplantation is the preferred treatment for most patients with chronic organ failure. Despite great improvement in treatments to inhibit rejection, rejection continues to be the single largest impediment to successful organ transplantation. Rejection includes not only acute rejection but also chronic rejection. One-year survival rates for transplanted kidneys average 88.3% with kidneys from deceased donors and 94.4% with kidneys received from living donors. The corresponding five year survival rates for the transplanted kidneys are 63.3% and 76.5% (OPTN/SRTR Annual Report, 2002). For livers the one year survival rates are 80.2% and 76.5% for livers from deceased and living donors, respectively. The corresponding five year liver graft survival rates are 63.5% and 73.0% (OPTN/SRTR Annual Report, 2002). The use of immunosuppressant drugs, especially cyclosporin A and more recently tacrolimus, has dramatically improved the success rate of organ transplantation especially by preventing acute rejection. But as the numbers above show, there is still a need to improve the success rates, both short-term and especially long-term. As seen from the above numbers for kidney and liver transplants, the five year failure rates for these transplanted organs are on the order of 25-35%. In the year 2001 alone there were more than 23,000 patients who received an organ transplant of which approximately 19,000 were kidney or liver (OPTN/SRTR Annual Report, 2002). For this one year of transplants alone, with present techniques it can be expected that approximately 5,000-6,000 of these transplanted kidneys and livers will fail within 5 years. These numbers do not even include other transplanted organs or transplanted tissues or cells such as bone marrow.

There are multiple types of transplants. These are described in Abbas et al., 2000. A graft transplanted from one individual to the same individual is called an autologous graft or autograft. A graft transplanted between two genetically identical or syngeneic individual is called a syngeneic graft. A graft transplanted between two genetically different individuals of the same species is called an allogeneic graft or allograft. A graft transplanted between individuals of different species is called a xenogeneic graft or xenograft. The molecules that are recognized as foreign on allografts are called alloantigens and those on xenografts are called xenoantigens. The lymphocytes or antibodies that react with alloantigens or xenoantigens are described as being alloreactive or xenoreactive, respectively.

Currently more than 40,000 kidney, heart, lung, liver and pancreas transplants are performed in the United States each year (Abbas et al., 2000). Other possible transplants include, but are not limited to, vascular tissue, eye, cornea, lens, skin, bone marrow, muscle, connective tissue, gastrointestinal tissue, nervous tissue, bone, stem cells, islets, cartilage, hepatocytes, and hematopoietic cells. Unfortunately, there are many more candidates for a transplant than there are donors. To overcome this shortage, a major effort is being made to learn how to use xenografts. While progress is being made in this field, the fact is that at present most transplants are allografts. An allogeneic transplant, while presently being more likely to be successful than a xenogeneic transplant, must surmount numerous obstacles to be successful. There are several types of immunological attacks made by the recipient against the donor organ which can lead to rejection of the allograft. These include hyperacute rejection, acute vascular rejection (including accelerated humoral rejection and de novo acute humoral rejection), and chronic rejection. Rejection is normally a result of T-cell mediated or humoral antibody attack, but may include additional secondary factors such as the effects of complement and cytokines.

An ever growing gap between the number of patients requiring organ transplantation and the number of donor organs available has become a major problem throughout the world. Park et al., 2003. Individuals who have developed anti-HLA antibodies are said to be immunized or sensitized. Gloor, 2005. HLA sensitization is the major barrier to optimal utilization of organs from living donors in clinical transplantation (Warren et al., 2004) due to the development of severe antibody-mediated rejection (ABMR). For example, more than 50% of all individuals awaiting kidney transplantation are presensitized patients (Glotz et al., 2002) who have elevated levels of broadly reactive alloantibodies, resulting from multiple transfusions, prior failed allografts, or pregnancy (Kupiec-Weglinski, 1996). The role of ABMR is currently one of the most dynamic areas of study in transplantation, due to recognition that this type of rejection can lead to either acute or chronic loss of allograft function. Mehra et al., 2003. Numerous cases of ABMR, including hyperacute rejection (HAR) or accelerated humoral rejection (ACHR), have been reported that are characterized by acute allograft injury that is resistant to potent anti-T cell therapy, the detection of circulating donor specific antibodies, and the deposition of complement components in the graft. ABMR with elevated circulating alloantibodies and complement activation that occurs in 20-30% of acute rejection cases has a poorer prognosis than cellular rejection. Mauiyyedi et al., 2002.

Highly presensitized patients, who exhibit high levels of alloantibodies, usually suffer an immediate and aggressive HAR. In clinical practice, with great efforts and significant advances in technology, HAR is avoided by obtaining a pretransplant lymphocytotoxic crossmatch to identify sensitized patients with antibodies specific for donor HLA antigens. However, circulating antibodies against donor HLA or other non-MHC endothelial antigens may also be responsible for a delayed form of acute humoral rejection, which is associated with an increased incidence of graft loss. Collins et al., 1999. Therefore, development of a novel presensitized animal model to mimic ABMR in clinical settings would be beneficial to studies on the mechanism, and to the much needed progress in the management of allograft rejection in presensitized hosts.

Some highly presensitized patients can benefit from intervention programs such as immunoadsorption (Palmer et al.,1989; Ross et al., 1993; Kriaa et al., 1995), plasmapheresis and intravenous immunoglobulin (Sonnenday et al., 2002; Rocha et al., 2003), that have been designed and implemented to temporarily eliminate anti-donor antibodies. However, in addition to their benefits, the aforementioned therapies carry with them numerous drawbacks as some individuals are less susceptible to their effects (Kriaa et al., 1995; Hakim et al., 1990; Glotz et al., 1993; Tyan et al., 1994); they are extremely expensive, time-consuming, and risky (Salama et al., 2001). Moreover, the transient and variable effect of these protocols has limited their impact. Glotz et al., 2002; Kupin et al., 1991; Schweitzer et al., 2000. Therefore, developing novel strategies to reduce the risk and cost in prevention of ABMR would be beneficial to presensitized recipients receiving an allograft.

SUMMARY

Accordingly, methods of prolonging survival of transplanted cells, tissues or organs are provided. In particular, methods of prolonging survival of allotransplanted cells, tissues or organs are provided. These methods are directed to using one or more immunosuppressants in addition to an inhibitor of complement activity. Use of one or more immunosuppressants and an inhibitor of complement activity in the manufacture of one or more medicaments or medicament packages is also provided. Such medicaments or medicament packages are useful in prolonging survival of an allograft in a subject mammal.

In certain embodiments, the inhibition of complement activity is effected by chronic administration of a drug directed against complement C5. A preferred drug that inhibits complement activity is an antibody specific to one or more components of complement, for example, C5. In certain preferred embodiments, the antibody inhibits the cleavage of C5 and thereby inhibits the formation of both C5a and C5b-9. The antibody may be, e.g., a monoclonal antibody, a chimeric antibody (e.g., a humanized antibody), an antibody fragment (e.g., Fab), a single chain antibody, an Fv, or a domain antibody. The recipient is also treated with one or more immunosuppressive drugs, for example, cyclosporin A.

In certain embodiments, either an MHC mismatched recipient (i.e., a mammalian recipient of an MHC mismatched allograft), a presensitized recipient or an ABO mismatched recipient (i.e., a mammalian recipient of an AMB mismatched allograft) is treated. In this model, the recipient is again chronically treated with a complement inhibitor, preferably an anti-C5 monoclonal antibody, together with immunosuppressive drugs, preferably a chronic administration of cyclosporin A and a short-term administration of cyclophosphamide. This triple therapy results in extended graft survival in the presensitized allotransplant recipient.

The present disclosure also provides methods of prolonging survival of an allograft in a mammalian recipient by administering to the recipient agents that modulate the level and/or ratio of subclasses and/or isotypes of anti-donor immunoglobulins (Ig) in the recipient. In certain embodiments, an agent that reduces the level of anti-donor IgG1 in the recipient is preferred. In certain embodiments, an agent that increases the level of anti-donor IgG2a and/or IgG2b in the recipient is preferred. In certain embodiments, an agent that reduces the ratio of anti-donor IgG1/anti-donor IgG2a or IgG2b in the recipient is preferred.

The present disclosure also provides a method of prolonging survival of an allograft in a second mammalian recipient using an allograft that has been accommodated in a first mammalian recipient (i.e., the allograft has prolonged survival in the first recipient). The present disclosure further provides an allograft that is resistant to anti-donor antibodies in a mammalian recipient, and the allograft is prepared from a first recipient that has accommodated the allograft. In preferred embodiments, the first recipient has accommodated the allograft by receiving a treatment as described herein, such a triple therapy treatment involving administering to the first recipient a drug that inhibits complement activity and two immunosuppressive agents.

Further provided are pharmaceutical packages. A pharmaceutical package of the present disclosure may comprise a drug that inhibits complement activity and at least one immunosuppressive agent. The pharmaceutical package may further comprise a label for chronic administration. The pharmaceutical package may also comprise a label for self-administration by a patient, for example, a recipient of a transplant graft, or instructions for a caretaker of a recipient of a transplant graft. In certain embodiments, the drug and the agent in the pharmaceutical package are in a formulation or separate formulations that are suitable for chronic administration and/or self-administration.

The present disclosure also provides lyophilized formulations and formulations suitable for injection. Certain embodiments provide a lyophilized antibody formulation comprising an antibody that inhibits complement activity and a lyoprotectant. In preferred embodiments, the antibody formulation is suitable for chronic administration, for example, the antibody formulation is stable. Alternative embodiments provide an injection system comprising a syringe; the syringe comprises a cartridge containing an antibody that inhibits complement activity and is in a formulation suitable for injection.

An antibody employed in various embodiments of the present disclosure preferably inhibits the formation of terminal complement or C5a. In certain embodiments, antibody inhibits formation of terminal complement or C5a is a whole antibody or an antibody fragment. The whole antibody or antibody fragment may be a human, humanized, chimerized or deimmunized antibody or antibody fragment. In certain embodiments, the whole antibody or antibody fragment may inhibit cleavage of complement C5. In certain embodiments, the antibody fragment is a Fab, an F(ab')2, an Fv, a domain antibody, or a single-chain antibody. In preferred embodiments, the antibody fragment is pexelizumab. In alternative preferred embodiments, the whole antibody is eculizumab.

In certain embodiments, a drug, such as an antibody, that inhibits complement activity is present in unit dosage form, which can be particularly suitable for self-administration. Similarly, an immunosuppressive agent of the present disclosure may also be present in unit dosage form.

BRIEF DESCRIPTION OF THE DRAWINGS

Figures 1A-1D show anti-donor antibody levels in presensitized versus unsensitized recipients under different treatments.

Figures 2A and 2B show comparison between triple therapy using anti-C5 antibody, CsA and CyP in presensitized allograft recipients and combination therapy using only anti-C5 antibody and CsA in presensitized allograft recipients. Figure 2A compares heart-allograft survival in various recipients under different treatments as indicated. Figure 2B shows histology and immunohistology, for example, for lymphocyte infiltration in heart allografts of recipients in different groups.

Figure 3 shows blocked terminal complement activity by anti-C5 antibody as compared to immunosuppressive agents.

Figures 4A-4D compare levels of anti-donor antibodies in presensitized recipients of allografts under monotherapy with anti-C5 antibody alone, double combination therapy with anti-C5 antibody and CsA, and triple combination therapy with anti-C5 antibody, CsA and CyP.

Figures 5A and 5B show change of ratios of IgG isotypes in allograft recipients that were untreated or under different treatments.

Figure 6 shows high-level expression of Bcl-2 and Bcl-xl proteins in long-term surviving heart grafts as compared to heart grafts of untreated animals.

Figure 7 shows improved second transplantation (re-transplantation) of an accommodated graft from a first transplantation recipient.

Figure 8 shows results from re-transplantation experiments.

Figure 9 shows results from re-transplantation experiments.

DETAILED DESCRIPTION

*Overview: Rejection of Transplants or Grafts*

Hyperacute rejection occurs within minutes to hours after transplant and is due to preformed antibodies to the transplanted tissue antigens. It is characterized by hemorrhage and thrombotic occlusion of the graft vasculature. The binding of antibody to endothelium activates complement, and antibody and complement induce a number of changes in the graft endothelium that promote intravascular thrombosis and lead to vascular occlusion, the result being that the grafted organ suffers irreversible ischemic damage (Abbas et al., 2000). Hyperacute rejection is often mediated by preexisting IgM alloantibodies, e.g., those directed against the ABO blood group antigens expressed on red blood cells. This type of rejection, mediated by natural antibodies, is the main reason for rejection of xenotransplants. Hyperacute rejection due to natural IgM antibodies is no longer a major problem with allografts because allografts are usually selected to match the donor and recipient ABO type. Hyperacute rejection of an ABO matched allograft may still occur, usually mediated by IgG antibodies directed against protein alloantigens, such as foreign MHC molecules, or against less well defined alloantigens expressed on vascular endothelial cells. Such antibodies may arise as a result of prior exposure to alloantigens through blood transfusion, prior transplantation, or multiple pregnancies (this prior exposure being referred to as "presensitization"). Abbas et al., 2000.

Acute rejection is a process of vascular and parenchymal injury mediated by T cells, macrophages, and antibodies that usually begins after the first week of transplantation. Abbas et al., 2001. T lymphocytes play a central role in acute rejection by responding to alloantigens, including MHC molecules, present on vascular endothelial and parenchymal cells. The activated T cells cause direct lysis of graft cells or produce cytokines that recruit and activate inflammatory cells, which cause necrosis. Both $CD4^+$ and $CD8^+$ cells may contribute to acute rejection. The destruction of allogeneic cells in a graft is highly specific and a hallmark of $CD8^+$ cytotoxic T lymphocyte killing. Abbas et al., 2000. $CD4^+$ T cells may be important in mediating acute graft rejection by secreting cytokines and inducing delayed-type hypersensitivity-like reactions in grafts, with some evidence available that indicates that $CD4^+$ T cells are sufficient to mediate acute rejection. Abbas et al., 2000. Antibodies can also mediate acute rejection after a graft recipient mounts a humoral immune response to vessel wall antigens and the antibodies that are produced bind to the vessel wall and activate complement. Abbas et al., 2000.

Chronic rejection is characterized by fibrosis with loss of normal organ structures occurring over a prolonged period. The pathogenesis of chronic rejection is less well understood than that of acute rejection. Graft arterial occlusion may occur as a result of the proliferation of intimal smooth muscle cells (Abbas et al., 2000). This process is called accelerated or graft arteriosclerosis and can develop in any vascularized organ transplant within 6 months to a year after transplantation.

For a transplant to be successful, the several modes of rejection must be overcome. Multiple approaches are utilized in preventing rejection. This may require administration of immunosuppressants, often several types to prevent the various modes of attack, e.g., inhibition of T-cell attack, antibodies, and cytokine and complement effects. Prescreening of donors to match them with recipients is also a major factor in preventing rejection, especially in preventing hyperacute rejection. Immunoadsorption of anti-HLA antibodies prior to grafting may reduce hyperacute rejection. Prior to transplantation the recipient or host may be administered anti-T cell reagents, e.g., the monoclonal antibody OKT3, Anti-Thymocyte Globulin (ATG), cyclosporin A, or tacrolimus (FK 506). Additionally, glucocorticoids and/or azathioprine may be administered to the host prior to transplant. Drugs used to aid in preventing transplant rejection include, but are not limited to, ATG or ALG, OKT3, daclizumab, basiliximab, corticosteroids, 15-deoxyspergualin, cyclosporins, tacrolimus, azathioprine, methotrexate, mycophenolate mofetil, 6-mercaptopurine, bredinin, brequinar, leflunamide, cyclophosphamide, sirolimus, anti-CD4 monoclonal antibodies, CTLA4-Ig, anti-CD 154 monoclonal antibodies, anti-LFA1 monoclonal antibodies, anti-LFA-3 monoclonal antibodies, anti-CD2 monoclonal antibodies, and anti-CD45.

Allografts are rejected in part by the activation of T cells. The transplant recipient mounts a rejection response following $CD4^+$ T cell recognition of foreign antigens in the allograft. These antigens are encoded by the major histocompatibility complex (MHC). There are both Class I and Class II MHC molecules. In humans the class I MHC molecules are HLA-A, HLA-B, and HLA-C. The class II MHC molecules in humans are called HLA-DR, HLA-DQ and HLA-DP. In mice the class I MHC molecules are H-2K, H-2D and H-2L and the class II MHC molecules are I-A and I-E. When $CD4^+$ T cells bind the foreign MHC antigens they are activated and undergo clonal proliferation. The activated T cells secrete cytokines which aid in activating monocytes/macrophages, B cells and cytotoxic $CD8^+$ T cells. The activated monocytes/macrophages release agents which result in tissue damage, the B cells produce alloantibodies which lead to complement mediated tissue destruction, and the $CD8^+$ T cells kill graft cells in an antigen-specific manner through induction of apoptosis and cell lysis.

*Immunosuppressive Agents*

The numerous drugs utilized to delay graft rejection (i.e., to prolong their survival) work in a variety of ways. Immunosuppressive agents are widely used. See Stepkowski, 2000, for a review of the mechanism of action of several immunosuppressive drugs. Cyclosporin A is one of the most widely used immunosuppressive drugs for inhibiting graft rejection. It is an inhibitor of interleukin-2 or IL-2 (it prevents mRNA transcription of interleukin-2). More directly, cyclosporin inhibits calcineurin activation that normally occurs upon T cell receptor stimulation. Calcineurin dephosphorylates NFAT (nuclear factor of activated T cells) enabling it to enter the nucleus and bind to interleukin-2 promoter. By blocking this process, cyclosporin A inhibits the activation of the CD4$^+$ T cells and the resulting cascade of events which would otherwise occur. Tacrolimus is another immunosuppressant that acts by inhibiting the production of interleukin-2.

Rapamycin (Sirolimus), SDZ RAD, and interleukin-2 receptor blockers are drugs that inhibit the action of interleukin-2 and therefore prevent the cascade of events described above.

Inhibitors of purine or pyrimidine biosynthesis are also used to inhibit graft rejection. These prevent DNA synthesis and thereby inhibit cell division including the ability of T cells to divide. The result is the inhibition of T cell activity by preventing the formation of new T cells. Inhibitors of purine synthesis include azathioprine, methotrexate, mycophenolate mofetil (MMF) and mizoribine (bredinin). Inhibitors of pyrimidine synthesis include brequinar sodium and leflunomide. Cyclophosphamide is an inhibitor of both purine and pyrimidine synthesis.

Yet another method for inhibiting T cell activation is to treat the recipient with antibodies to T cells. OKT3 is a murine monoclonal antibody against CD3 which is part of the T cell receptor. This antibody inhibits the T cell receptor and suppresses T cell activation.

Numerous other drugs and methods for delaying allotransplant rejection are known to and used by those of skill in the art. One approach has been to deplete T cells, e.g., by irradiation. This has often been used in bone marrow transplants, especially if there is a partial mismatch of major HLA. Administration to the recipient of an inhibitor (blocker) of the CD40 ligand-CD40 interaction and/or a blocker of the CD28-B7 interaction has been used (U.S. Patent 6,280,957). Published PCT patent application WO 01/37860 teaches the administration of an anti-CD3 monoclonal antibody and IL-5 to inhibit the Thl immune response. Published PCT patent application WO 00/27421 teaches a method for prophylaxis or treatment of corneal transplant rejection by administering a tumor necrosis factor-α antagonist. Glotz et al. (2002) show that administration of intravenous immunoglobulins (IVIg) can induce a profound and sustained decrease in the titers of anti-HLA antibodies thereby allowing a transplant of an HLA-mismatched organ. Similar protocols have included plasma exchanges (Taube et al., 1984) or immunoadsorption techniques coupled to immunosuppressive agents (Hiesse et al., 1992) or a combination of these (Montgomery et al., 2000). Changelian et al. (2003) teach a model in which immunosuppression is caused by an oral inhibitor of Janus kinase 3 (JAK3) which is an enzyme necessary for the proper signaling of cytokine receptors which use the common gamma chain (yc) (Interleukins-2, -4, -7, -9, -15, -21), the result being an inhibition of T cell activation. Antisense nucleic acids against ICAM-1 have been used alone or in combination with a monoclonal antibody specific for leukocyte-function associated antigen 1 (LFA-1) in a study of heart allograft transplantation (Stepkowski, 2000). Similarly, an anti-ICAM-1 antibody has been used in combination with anti-LFA-1 antibody to treat heart allografts (Stepkowski, 2000). Antisense oligonucleotides have additionally been used in conjunction with cyclosporin in rat heart or kidney allograft models, resulting in a synergistic effect to prolong the survival of the grafts (Stepkowski, 2000). Chronic transplant rejection has been treated by administering an antagonist of TGF-β which is a cytokine involved in differentiation, proliferation and apoptosis (U.S. Patent Application Publication US 2003/0180301).

*Complement and Transplant/Graft Rejection*

The role of complement in transplant rejection is well known. This is especially true in the case of xenotransplantation, but complement also plays a role in allotransplant rejection. For review, see Platt and Saadi, 1999. One aspect of complement's role is that ischemia-reperfusion injury may occur at the time that an organ graft is reperfused with the blood of the recipient. Complement may also cause some manifestations of allograft rejection.

The complement system is described in detail in U.S. Patent 6,355,245. The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions.

The complement cascade progresses via the classical pathway or the alternative pathway. These pathways share many components and, while they differ in their initial steps, they converge and share the same "terminal complement" components (C5 through C9) responsible for the activation and destruction of target cells.

The classical complement pathway is typically initiated by antibody recognition of and binding to an antigenic site on a target cell. The alternative pathway is usually antibody independent and can be initiated by certain molecules on pathogen surfaces. Both pathways converge at the point where complement component C3 is cleaved by an active protease (which is different in each pathway) to yield C3a and C3b. Other pathways activating complement attack can act later in the sequence of events leading to various aspects of complement function.

C3a is an anaphylatoxin. C3b binds to bacterial and other cells, as well as to certain viruses and immune complexes, and tags them for removal from the circulation. C3b in this role is known as opsonin. The opsonic function of C3b is considered to be the most important anti-infective action of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to Neisseria infection, and then only somewhat more prone (Fearon, 1983).

C3b also forms a complex with other components unique to each pathway to form classical or alternative C5 convertase, which cleaves C5 into C5a and C5b. C3 is thus regarded as the central protein in the complement reaction sequence since it is essential to both the alternative and classical pathways (Wurzner et al., 1991). This property of C3b is regulated by the serum protease Factor I, which acts on C3b to produce iC3b. While still functional as opsonin, iC3b cannot form an active C5 convertase.

C5 is a 190 kDa beta globulin found in normal serum at approximately 75 $\mu$g/mL (0.4 $\mu$M). C5 is glycosylated, with about 1.5-3 percent of its mass attributed to carbohydrate. Mature C5 is a heterodimer of a 999 amino acid 115 kDa alpha chain that is disulfide linked to a 656 amino acid 75 kDa beta chain. C5 is synthesized as a single chain precursor protein product of a single copy gene (Haviland et al., 1991). The cDNA sequence of the transcript of this gene predicts a secreted pro-C5 precursor of 1659 amino acids along with an 18 amino acid leader sequence.

The pro-C5 precursor is cleaved after amino acid 655 and 659, to yield the beta chain as an amino terminal fragment (amino acid residues +1 to 655) and the alpha chain as a carboxyl terminal fragment (amino acid residues 660 to 1658), with four amino acids deleted between the two.

C5a is cleaved from the alpha chain of C5 by either alternative or classical C5 convertase as an amino terminal fragment comprising the first 74 amino acids of the alpha chain (i.e., amino acid residues 660-733). Approximately 20 percent of the 11 kDa mass of C5a is attributed to carbohydrate. The cleavage site for convertase action is at or immediately adjacent to amino acid residue 733. A compound that would bind at or adjacent to this cleavage site would have the potential to block access of the C5 convertase enzymes to the cleavage site and thereby act as a complement inhibitor.

C5 can also be activated by means other than C5 convertase activity. Limited trypsin digestion (Minta and Man, 1977; Wetsel and Kolb, 1982) and acid treatment (Yamamoto and Gewurz, 1978; Vogt et al., 1989) can also cleave C5 and produce active C5b.

C5a is another anaphylatoxin. C5b combines with C6, C7, and C8 to form the C5b-8 complex at the surface of the target cell. Upon binding of several C9 molecules, the membrane attack complex (MAC, C5b-9, terminal complement complex-TCC) is formed. When sufficient numbers of MACs insert into target cell membranes the openings they create (MAC pores) mediate rapid osmotic lysis of the target cells. Lower, non-lytic concentrations of MACs can produce other effects. In particular, membrane insertion of small numbers of the C5b-9 complexes into endothelial cells and platelets can cause deleterious cell activation. In some cases activation may precede cell lysis.

As mentioned above, C3a and C5a are anaphylatoxins. These activated complement components can trigger mast cell degranulation, which releases histamine and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract pro-inflammatory granulocytes to the site of complement activation.

Complement-binding recipient antibodies to donor alloantigens are considered to be the main cause of hyperacute graft rejection. Owing to pretransplant crossmatch testing, this prototype of humoral rejection is now rarely observed (Regele et al., 2001). Data are now showing that humoral immune mechanisms might contribute to other types of allograft rejection (Regele et al., 2001). High levels of panel reactive antibodies indicating humoral presensitization were found to be associated with inferior kidney graft survival (Opelz, 1992), the appearance of alloantibodies during the post-transplant period has been reported to predict poor graft outcome (Jeannet et al., 1970; Halloran et al., 1992), and selective removal of recipient IgG by immunoadsorption reversed some rejection episodes indicating the contribution of humoral immune mechanisms to rejection (Persson et al., 1995; Böhmig et al., 2000). Complement activation within a graft might indicate antibody-mediated graft injury. The complement cleavage product C4d is a marker for activation of the antibody-dependent classical pathway. Capillary C4d deposits in kidney allograft biopsies were associated with poor graft outcome.

Recently increasing evidence indicates that complement activation significantly contributes to the sensitization of allograft recipients and the development of tissue injury in allografts (Platt et al., 1999). Antibodies are the most thoroughly investigated mediators of activating the classical complement pathway. Clinically, alloantibodies are known to activate complement (Baldwin et al., 2001). Halloran and Collins indicate that C4d deposition in peritubular capillaries of renal allografts is a sensitive and diagnostic marker of acute humoral rejection that correlates strongly with the presence of circulating donor-specific antibodies (Collins et al., 1999; Halloran, 2003). Further supporting evidence is seen in animals with complement inhibition (Pratt et al., 1996; Pruitt et al., 1991; Forbes et al., 1978) or deficiency (Pratt et al., 2000; Baurer et al., 1995) which exhibit significantly reduced inflammatory injury and lowered anti-donor immune responses. In ABMR, complement is suggested to be activated by the classical pathway and to play a key role in the pathogenesis (Collard et al., 1997). Although the role of complement in HAR or acute vascular rejection (AVR) following xenotransplantation has been well documented (Platt et al., 1999), precise mechanisms of complement in the pathogenesis of ABMR following allotransplantation has not yet been elucidated.

The C5 component of complement is cleaved to form products with multiple proinflammatory effects and thus represents an attractive target for complement inhibition within the immune-mediated inflammatory response. As described above, C5a is a powerful anaphylatoxin and chemotactic factor. Cellular activation by C5a induces the release of multiple additional inflammatory mediators (Jose et al., 1983). The complement activation pathways (classical, alternative, or mannan-binding lectin pathway) ultimately lead to the formation of the cytolytic membrane attack complex C5b-9 (Kirschfunk, 2001), which can mediate both direct tissue injury by cell lysis, and proinflammatory cell activation at sublytic doses (Saadi et al., 1995; Papadimitriou et al., 1991). Therefore, blocking both C5a and C5b-9 generation may be required for the optimal inhibition of complement-mediated inflammatory response following transplantation. At the same time, inhibition of the complement cascade at C5 does not impair the generation of C3b, preserving C3b-mediated opsonization of pathogenic microorganisms as well as solubilization and clearance of immune complexes (Liszewski, 1993).

The beneficial effect of anti-C5 mAb has previously been reported in several experimental models including myocardial reperfusion (Vakeva et al., 1998), systemic lupus erythematosus (Wang et al., 1996) and rheumatoid arthritis (Wang et al., 1995); as well as in human clinical trials (Kirschfink, 2001) of autoimmune disease, cardiopulmonary bypass and acute myocardial infarction. In addition, complement inactivation by a functionally blocking anti-C5 monoclonal Ab (mAb) prevented HAR in xenotransplantation models (Kroshus et al., 1995; Wang et al., 1999).

Methods of delaying allotransplant rejection by administration of complement inhibitors have been tested. Published PCT patent application WO 92/10205 discloses the use of a combination of cyclosporin and a soluble complement receptor (sCR1) to inhibit rejection of a cardiac allotransplant in a presensitized rat model. Complement receptor 1 binds complements C3b and C4b. Soluble forms of complement receptor 1 occur naturally or can be generated via recombinant DNA procedures. These soluble complement receptors have inhibited in vitro the consequences of complement activation (U.S. Patent 6,057,131). In WO 92/10205, rats, which had been presensitized to the cardiac allograft they were receiving, were administered cyclosporin A intramuscularly at 10 mg/kg/day beginning two days prior to transplant and continued until the time of graft rejection. Additionally, soluble complement receptor 1 (sCR1) was administered as a single intravenous bolus at 15 mg/kg immediately prior to reperfusion of the graft. Control animals with no drug treatment had the graft rejected at an average of 3.8 days. Those administered cyclosporin A alone rejected the grafts at an average of 57 days (this was quite variable with two rats rejecting quickly at 2 and 4 days and a third rat rejecting at 166 days). Rats administered sCR1 alone rejected the grafts at an average of 44 days. Those rats administered the combination of cyclosporin A and sCR1 rejected the grafts at an average of 147 days. The combination of chronic cyclosporin A and single bolus sCR1 was seen to result in a synergistic effect greatly prolonging the time until graft rejection. Earlier studies by Pruitt and Bollinger (1991) used a similar model of a presensitized rat allograft to show that administration of sCR1 alone to inactivate complement resulted in increased time before graft rejection.

Sims et al. (U.S. Patent 5,135,916) suggest using inhibitors of complement, e.g., CD59 or antibodies against C7 or C9 to block the formation of the C5b-9 complex, to treat the vascular endothelium of organs and tissues to be transplanted. This would prevent the C5b-9 initiated cell necrosis. The C5b-9 inactivators would be added to the perfusate or storage medium to protect the vascular lining cells from ongoing complement activation during in vitro storage. Additionally the organ or tissue would be protected from the cytolytic and thrombotic effects arising from complement activation initiated upon transplantation, thereby circumventing complement mediated acute rejection. Sims et al. (U.S. Patent 5,573,940 and U.S. Patent 6,100,443) also teach a method of expressing CD59 in the transplanted tissue or organ to protect the transplanted organ from rejection. This can be accomplished by transfecting the cells being transplanted.

Although the several drugs developed to date in combination with methods of prescreening donors and recipients to match the donor allograft to the recipient have over time increased the average length of time of survival of allografts, many allografts are nonetheless rejected during the life-time of the recipient. In general, the prior art advances have mainly been directed to overcoming acute graft rejection. Further, the role of activated terminal complement components in antibody-mediated allograft rejection has not been examined using inhibitors that specifically target the complement cascade at the C5 protein level. The methods described herein and as exemplified in the Examples advance the allotransplant art by inhibiting chronic rejection of allografts, in particular, allografts in a presensitized recipient. New methods are presented for further prolonging allograft survival by using a proper combination of immunosuppressive drugs in combination with a chronic administration of a complement inhibitor.

*Methods and Uses*

The methods disclosed herein are used to prolong allograft survival. The methods generally include administering an inhibitor of complement activity in combination with one or more immunosuppressants.

Suitable complement inhibitors are known to those of skill in the art. Antibodies can be made to individual components of activated complement, e.g., antibodies to C5a, C7, C9, etc. (see, e.g., U.S. Patent 6,534,058; published U.S. patent application US 2003/0129187; and U.S. Patent 5,660,825). Proteins are known which inhibit complement-mediated lysis, including CD59, CD55, CD46 and other inhibitors of C8 and C9 (see, e.g., U.S. Patent 6,100,443). U.S. Patent 6,355,245 teaches an antibody which binds to C5 and prevents it from being cleaved into C5a and C5b thereby preventing the formation not only of C5a but also the C5b-9 complex. Proteins known as complement receptors and which bind complement are also known (see, Published PCT Patent Application WO 92/10205 and U.S. Patent 6,057,131). Use of soluble forms of complement receptors, e.g., soluble CR1, can inhibit the consequences of complement activation such as neutrophil oxidative burst, complement mediated hemolysis, and C3a and C5a production. Those of skill in the art recognize the above as some, but not all, of the known methods of inhibiting complement and its activation.

Suitable immunosuppressants include, but are not limited to, ATG or ALG, OKT3, daclizumab, basiliximab, corticosteroids, 15-deoxyspergualin, cyclosporins, tacrolimus, azathioprine, methotrexate, mycophenolate mofetil, 6-mercaptopurine, bredinin, brequinar, leflunamide, cyclophosphamide, sirolimus, anti-CD4 monoclonal antibodies, CTLA4-Ig, anti-CD154 monoclonal antibodies, anti-LFA1 monoclonal antibodies, anti-LFA-3 monoclonal antibodies, anti-CD2 monoclonal antibodies, and anti-CD45.

An allograft can include a transplanted organ, part of an organ, tissue or cell. These include, but are not limited to, heart, kidney, lung, pancreas, liver, vascular tissue, eye, cornea, lens, skin, bone marrow, muscle, connective tissue, gastrointestinal tissue, nervous tissue, bone, stem cells, islets, cartilage, hepatocytes, and hematopoietic cells.

At least part of the reason for the failure of allografts is that one response by the recipient of an allograft is the activation of complement. This results in the formation of C5a and C5b-9 which are potent proinflammatory molecules which aid in causing graft failure. Without wishing to be bound by any proposed theory, Applicants theorized that inhibiting the formation of C5a and C5b-9 or inhibiting C5a and C5b-9 which was present would aid in preventing graft failure. Furthermore, it was theorized that so long as the allograft is present, the recipient will continue to attempt to mount an immune response against the graft, and this response will include attempts to produce C5a and C5b-9. If not prevented, this complement response will lead to acute vascular rejection in the short term and could contribute to chronic graft rejection in the long term. Prior art methods of using inhibitors of complement activity were limited to administering these inhibitors only at the time of transplant. This was helpful in preventing acute rejection, but as the results disclosed herein illustrate, improved results are obtained by administration of such inhibitors for a longer term. This long-term administration aids in preventing a chronic rejection of the allograft as opposed to only aiding in preventing an acute rejection. The result is a longer term survival of the allograft as compared to either not administering an inhibitor of complement activity or administering such an inhibitor only at the time of transplant of the allograft. Although very commonly it is desirable that the allograft will survive for the remaining lifetime of the recipient, there are times when the allograft is needed only for a shorter length of time, e.g., a bridge organ to bridge the time until the recipient's own organ can recover on its own, at which time the allograft will no longer be needed. The length of time such a graft will be needed will vary, but will usually be longer than the time at which acute rejection would occur and may be long enough for chronic rejection to occur. This period of desired survival for a bridge graft may be several months, e.g., six months.

To prove that long-term inhibition of complement activity will prolong allograft survival, experiments were performed in which complement activation was inhibited in a chronic fashion and not merely at the time of transplant. Chronic treatment means treatment during an extended period up to the lifetime of the allograft. This can be daily treatment but is not limited to daily treatment. Chronic treatment will maintain an effective amount of the drug in the allograft recipient. For example, a preferred method is to include the anti-C5 monoclonal antibody eculizumab in the treatment. In studies of persons suffering from paroxysmal nocturnal hemoglobinuria (PNH), eculizumab has been administered at a dose of 900 mg/patient once every 12-14 days. This dosing has been found to completely and consistently block terminal complement activity and has greatly inhibited the symptoms of PNH (Hillmen et al., 2004). The administered dose is able to block the effects of complement for approximately two weeks before the eculizumab is inactivated or removed from the body. Therefore, a chronic treatment of eculizumab may be, e.g., the administration of 900 mg to the allograft recipient once every two weeks for the remaining life-time of the patient. Similarly, other drugs can be delivered chronically as needed, whether this is on a daily basis or another schedule is required to maintain an effective amount of the drug in the allograft recipient. Because it is well known that graft rejection can be caused by more than just complement activation, e.g., by T cell activity, the experiments included immunosuppressants such as cyclosporin to further aid in preventing graft rejection.

A preferred method of inhibiting complement activity is to use a monoclonal antibody which binds to complement C5 and prevents C5 from being cleaved. This prevents the formation of both C5a and C5b-9 while at the same time allowing the formation of C3a and C3b which are beneficial to the recipient. Such antibodies that are specific to human complement are known (U.S. Patent 6,355,245). These antibodies disclosed in U.S. Patent 6,355,245 include both a whole or full-length antibody (now named eculizumab) and a single-chain antibody (now named pexelizumab). A similar antibody against mouse C5 is called BB5.1 (Frei et al., 1987). BB5.1 was utilized in the experiments set forth below. Antibodies to inhibit complement activity need not be monoclonal antibodies. They can be, e.g., polyclonal antibodies. They may additionally be antibody fragments. An antibody fragment includes, but is not limited to, an Fab, F(ab'), F(ab')$_2$, a single-chain antibody, a domain antibody and an Fv. Furthermore, it is well known by those of skill in the art that antibodies can be humanized (Jones et al., 1986), chimerized, or deimmunized. An antibody may also comprise a mutated Fc portion, such that the mutant Fc does not activate complement. The antibodies to be used in the present disclosure may be any of these. It is preferable to use humanized antibodies when the recipient of the allograft is a human.

*Administration and Formulations*

Administration of the inhibitor of complement activity is performed according to methods known to those of skill in the art. These inhibitors are administered preferably before the time of allograft transplantation or at the time of transplantation with administration continuing in a chronic fashion. These inhibitors can additionally be administered during a rejection episode in the event such an episode does occur.

The present disclosure also provides uses of a drug that inhibits complement activity and an immunosuppressive agent in the manufacture of a medicament or medicament package. Such medicament or medicament package is useful in prolonging allograft survival in a recipient, in particular, chronic survival of the allograft. In preferred embodiments, the medicament or medicament package is formulated and prepared such that it is suitable for chronic administration to the recipient of the allograft, for example, stable formulations are employed. In certain embodiments, the medicament or medicament package is formulated and prepared such that it is suitable for concurrent administration of the drug that inhibits complement activity and the immunosuppressive drug to the recipient of the allograft. In certain embodiments, the medicament or medicament package is formulated and prepared such that it is suitable for sequential (in either order) administration of the drug that inhibits complement activity and the immunosuppressive drug to the recipient of the allograft.

A pharmaceutical package of the present disclosure may comprise a drug that inhibits complement activity and at least one immunosuppressive agent. The pharmaceutical package may further comprise a label for chronic administration. The pharmaceutical package may also comprise a label for self-administration by a patient, for example, a recipient of a transplant graft, or instructions for a caretaker of a recipient of a transplant graft. In certain embodiments, the drug and the agent in the pharmaceutical package are in a formulation or separate formulations that are suitable for chronic administration and/or self-administration.

The present disclosure also provides lyophilized formulations and formulations suitable for injection. Certain embodiments provide a lyophilized antibody formulation comprising an antibody that inhibits complement activity and a lyoprotectant. In preferred embodiments, the antibody formulation is suitable for chronic administration, for example, the antibody formulation stable. Alternative embodiments provide an injection system comprising a syringe; the syringe comprises a cartridge containing an antibody that inhibits complement activity and is in a formulation suitable for injection.

An antibody employed in various embodiments of the present disclosure preferably inhibits the formation of terminal complement or C5a. In certain embodiments, antibody inhibits formation of terminal complement or C5a is a whole antibody or an antibody fragment. The whole antibody or antibody fragment may be a human, humanized, chimerized or deimmunized antibody or antibody fragment. In certain embodiments, the whole antibody or antibody fragment may inhibit cleavage of complement C5. In certain embodiments, the antibody fragment is a Fab, an F(ab')2, an Fv, a domain antibody, or a single-chain antibody. In preferred embodiments, the antibody fragment is pexelizumab. In alternative preferred embodiments, the whole antibody is eculizumab.

In certain embodiments, a drug, such as an antibody, that inhibits complement activity is present in unit dosage form, which can be particularly suitable for self-administration. Similarly, an immunosuppressive agent of the present disclosure may also be present in unit dosage form. A formulated product of the present disclosure can be included within a container, typically, for example, a vial, cartridge, prefilled syringe or disposable pen. A doser such as the doser device described in U.S. Patent 6,302,855 may also be used, for example, with an injection system of the present disclosure.

A "stable" formulation is one in which the drug (e.g., an antibody) or agent therein essentially retains its physical and chemical stability and integrity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993). Stability can be measured at a selected temperature for a selected time period. For example, the extent of aggregation following lyophilization and storage can be used as an indicator of protein stability. For example, a "stable" formulation may be one wherein less than about 10% and preferably less than about 5% of the protein is present as an aggregate in the formulation. In other embodiments, any increase in aggregate formation following lyophilization and storage of the lyophilized formulation can be determined. For example, a "stable" lyophilized formulation may be one wherein the increase in aggregate in the lyophilized formulation is less than about 5% and preferably less than about 3%, when the lyophilized formulation is stored at 2-8 °C for at least one year. In other embodiments, stability of the protein formulation may be measured using a biological activity assay.

A "reconstituted" formulation is one which has been prepared by dissolving a lyophilized protein formulation in a diluent such that the protein is dispersed in the reconstituted formulation. The reconstituted formulation in suitable for administration (e.g. parenteral administration) to a patient to be treated with the protein of interest and, in certain embodiments of the invention, may be one which is suitable for subcutaneous administration.

An isotonic reconstituted formulation is preferable in certain embodiments. By "isotonic" is meant that the formulation of interest has essentially the same osmotic pressure as human blood. Isotonic formulations will generally have an osmotic pressure from about 250 to 350 mOsm. Isotonicity can be measured using a vapor pressure or ice-freezing type osmometer, for example.

A "lyoprotectant" is a molecule which, when combined with a drug (e.g., antibody) of interest, significantly prevents or reduces chemical and/or physical instability of the drug (e.g., antibody) upon lyophilization and subsequent storage. Exemplary lyoprotectants include sugars such as sucrose or trehalose; an amino acid such as monosodium glutamate or histidine; a methylamine such as betaine; a lyotropic salt such as magnesium sulfate; a polyol such as trihydric or higher sugar alcohols, e.g. glycerin, erythritol, glycerol, arabitol, xylitol, sorbitol, and mannitol; propylene glycol; polyethylene glycol; Pluronics; and combinations thereof. The preferred lyoprotectant is a non-reducing sugar, such as trehalose or sucrose.

The lyoprotectant is added to the pre-lyophilized formulation in a "lyoprotecting amount" which means that, following lyophilization of the drug (e.g., antibody) in the presence of the lyoprotecting amount of the lyoprotectant, the drug (e.g., antibody) essentially retains its physical and chemical stability and integrity upon lyophilization and storage.

The "diluent" of interest herein is one which is pharmaceutically acceptable (safe and non-toxic for administration to a human) and is useful for the preparation of a reconstituted formulation. Exemplary diluents include sterile water, bacteriostatic water for injection (BWFI), a pH buffered solution (e.g. phosphate-buffered saline), sterile saline solution, Ringer's solution or dextrose solution.

A "preservative" is a compound which can be added to the diluent to essentially reduce bacterial action in the reconstituted formulation, thus facilitating the production of a multi-use reconstituted formulation, for example. Examples of potential preservatives include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl and benzyl alcohol, alkyl parabens such as methyl or propyl parahen, catechol, resorcinol, cyclohexanol, 3-pentanol, and m-cresol.

A "bulking agent" is a compound which adds mass to the lyophilized mixture and contributes to the physical structure of the lyophilized cake (e.g. facilitates the production of an essentially uniform lyophilized cake which maintains an open pore structure). Exemplary bulking agents include mannitol, glycine, polyethylene glycol and xorbitol.

Accordingly, a stable lyophilized antibody formulation can be prepared using a lyoprotectant (preferably a sugar such as sucrose or trehalose), which lyophilized formulation can be reconstituted to generate a stable reconstituted formulation having an antibody concentration which is significantly higher (e.g. from about 2-40 times higher, preferably 3-10 times higher and most preferably 3-6 times higher) than the antibody concentration in the pre-lyophilized formulation. Such high protein concentrations in the reconstituted formulation are considered to be particularly useful where the formulation is intended for subcutaneous administration. Despite the very high protein concentration in the reconstituted formulation, the reconstituted formulation can be stable (i.e. fails to display significant or unacceptable levels of chemical or physical instability of the protein) at 2-8 °C. for at least about 30 days. See U.S. Patent 6,821,515. In certain embodiments, the reconstituted formulation is isotonic.

When reconstituted with a diluent comprising a preservative (such as bacteriostatic water for injection, BWFI), the reconstituted formulation may be used as a multi-use formulation. Such a formulation is useful, for example, where a subject patient requires frequent administrations of the drug or antibody and/or agent to treat a chronic medical condition. The advantage of a multi-use formulation is that it facilitates ease of use for the patient, reduces waste by allowing complete use of vial contents, and results in a significant cost savings for the manufacturer since several doses are packaged in a single vial (lower filling and shipping costs).

The present disclosure also provides a method for preparing a formulation comprising the steps of: (a) lyophilizing a mixture of an antibody and a lyoprotectant; and (b) reconstituting the lyophilized mixture of step (a) in a diluent such that the reconstituted formulation is isotonic and stable.

An article of manufacture is also provided herein which comprises: (a) a container which holds a lyophilized mixture of an antibody and a lyoprotectant; and (b) instructions for reconstituting the lyophilized mixture with a diluent to a desirable antibody concentration in the reconstituted formulation. The article of manufacture may further comprise a second container which holds a diluent (e.g. bacteriostatic water for injection (BWFI) comprising an aromatic alcohol).

An injection system of the present disclosure may employ a medication delivery pen as described in U.S. Patent 5,308,341. Medication delivery pens have been developed to facilitate the self-administration of medication. A medication of the present disclosure can be a drug that inhibits complement activity, for example an antibody specific to complement C5, and/or an immunosuppressive agent. One medication delivery pen includes a vial holder into which a vial of insulin or other medication may be received. The vial holder is an elongate generally tubular structure with proximal and distal ends. The distal end of the vial holder includes mounting means for engaging a double-ended needle cannula. The proximal end also includes mounting means for engaging a pen body which includes a driver and dose setting apparatus. A disposable medication containing vial for use with the prior art vial holder includes a distal end having a pierceable elastomeric septum that can be pierced by one end of a double-ended needle cannula. The proximal end of this vial includes a stopper slidably disposed in fluid tight engagement with the cylindrical wall of the vial. This medication delivery pen is used by inserting the vial of medication into the vial holder. A pen body then is connected to the proximal end of the vial holder. The pen body includes a dose setting apparatus for designating a dose of medication to be delivery by the pen and a driving apparatus for urging the stopper of the vial distally for a distance corresponding to the selected dose.

The user of the pen mounts a double-ended needle cannula to the distal end of the vial holder such that the proximal point of the needle cannula pierces the septum on the vial. The patient then selects a dose and operates the pen to urge the stopper distally to deliver the selected dose. The dose selecting apparatus returns to zero upon injection of the selected dose. The patient then removes and discards the needle cannula, and keeps the prior art medication delivery pen in a convenient location for the next required medication administration. The medication in the vial will become exhausted after several such administrations of medication. The patient then separates the vial holder from the pen body. The empty vial may then be removed and discarded. A new vial can be inserted into the vial holder, and the vial holder and pen body can be reassembled and used as explained above.

Accordingly, a medication delivery pen generally has a drive mechanism for accurate dosing and ease of use. A dosage mechanism such as a rotatable knob allows the user to accurately adjust the amount of medication that will be injected by the pen from a prepackaged vial of medication. To inject the dose of medication, the user inserts the needle under the skin and depresses the knob once as far as it will depress. The pen may be an entirely mechanical device or it may be combined with electronic circuitry to accurately set and/or indicate the dosage of medication that is injected into the user. See U.S. Patent 6,192,891.

The present disclosure also presents controlled-release or extended-release formulations suitable for chronic and/or self-administration of a medication.

The various formulations can be administered to a patient in need of treatment (e.g., a recipient of an allograft) with the medication (e.g., an antibody of the present disclosure and at least one immunosuppressive agent) by intravenous administration as a bolus or by continuous infusion over a period of time, by intramuscular, intraperitoneal, intracerobrospinal, subcutaneous, intra-articular, intrasynovial, intrathecal, oral, topical, or inhalation routes.

In certain embodiments, a formulation is administered to the patient by subcutaneous (i.e. beneath the skin) administration. For such purposes, the formulation may be injected using a syringe. However, other devices for administration of the formulation are available such as injection devices (e.g. the Inject-ease® and Genject® devices); injector pens (such as the GenPen®); needleless devices (e.g. MediJector® and BioJector®); and subcutaneous patch delivery systems.

The present methods and uses are described with reference to the following Examples, which are offered by way of illustration and are not intended to limit the disclosure in any manner. Standard techniques well known in the art or the techniques specifically described below are utilized. The following abbreviations are used herein: ABMR, antibody-mediated rejection; ACHR, accelerated humoral rejection; ACR, acute cellular rejection; AVR, acute vascular rejection; CsA, cyclosporin; CyP, cyclophosphamide; HAR, hyperacute rejection; MCP-1, monocyte chemotactic protein 1; MST, mean survival time; POD, postoperative day.

Example 1

Methods

*Animals and Immunosuppressive Drugs* Male adult C3H (H-$2^k$) mice and BALB/c (H-$2^d$) mice (Jackson Labs, Bar Harbor, Maine) weighing 25-30 g were chosen as donors and recipients, respectively. In the groups receiving immunosuppression, the recipients were injected with CsA (15 mg/kg/day, s.c., daily from day 0 to endpoint rejection or until day 100), or with CyP (40 mg/kg/day, i.v., on day 0 and 1), or with anti-C5 mAb (clone BB5.1, Alexion Pharmaceuticals Inc., 40 mg/kg/day, i.p., day 0-2, followed by twice a week, day 0-60). Animals were housed under conventional conditions at the Animal Care Facility, University of Western Ontario, and were cared for in accordance with the guidelines established by the Canadian Council on Animal Care. Olfert et al., 1993.

*Skin Presensitization* Full-thickness skin grafts taken from C3H donors were cut into square pieces of 1×1 cm$^2$

and transplanted onto the back of the BALB/c recipients' thorax one week prior to heart transplantation from the same donors. Rejection was defined as complete necrosis of the skin grafts.

*Abdominal and Cervical Cardiac Transplantation* Seven days after skin presensitization, C3H mouse hearts were transplanted into the abdomen of presensitized BALB/c recipients by anastomosing the donor aorta and recipient aorta, and the donor pulmonary artery and recipient inferior vena cava. In the groups with re-transplantation, second heart grafts harvested from either naive C3H mice or long-term surviving presensitized BALB/c recipients were transplanted into the cervical area of the recipients carrying a long-term surviving first abdominal heart graft by anastomosing the donor aorta and recipient carotid artery, and the donor pulmonary artery and recipient external jugular vein (end-to-side). The heart grafts were monitored daily until rejection unless otherwise indicated and rejection was defined as complete cessation of pulsation.

*Experimental Groups* Presensitized recipients were randomly assigned to eight groups, each consisting of eight animals: Group 1, mice with no treatment; Group 2, mice treated with CsA; Group 3, mice treated with CyP; Group 4, mice treated with CsA plus CyP; Group 5, mice treated with anti-C5 mAb; Group 6, mice treated with anti-C5 mAb plus CsA; Group 7, mice treated with anti-C5 mAb plus CyP; Group 8, mice treated with anti-C5 mAb in combination of CsA and CyP. When cardiac impulses were no longer palpable or at POD100, the grafts were removed for routine histology, immunohistochemistry and western blot analysis, serum samples were collected for flow cytometric analysis and complement hemolytic assay. Five additional animals were placed and sacrificed in groups 6 and 8 on POD3 (MST for groups 1-5, 7) to allow for comparisons at a uniform time point. Serum samples were also collected on POD 11, 21, 28 and 60 in Group 8 for detecting the sequential changes of anti-donor antibody levels and complement activity. In addition, when triple therapy treated presensitized recipients carried a first heart graft for 100 days, they were re-transplanted with a second heart. A naive C3H heart or a 100-day surviving C3H heart from another presensitized BALB/c recipient was used as the second heart. Eight animals were included in each re-transplant group.

*Graft Histology* Tissue samples were fixed in 10% buffered formaldehyde. Specimens were then embedded in paraffin, and sectioned for H&E staining. The microscopic sections were examined in a blinded fashion for severity of rejection by a pathologist. Criteria for graft rejection included the presence of vasculitis, thrombosis, hemorrhage and lymphocyte infiltration. These changes were scored as: 0, no change; 1, minimum change; 2, mild change; 3, moderate change; or 4, marked change.

*Immunohistochemistry* Four micrometer sections were cut from tissue samples embedded in Tissue-Tek O.C.T gel (Optimum Cutting Temperature, Skura Finetek, Torrance, CA) mounted on gelatin-coated glass microscope slides and stained by a standard indirect avidin-biotin immunoperoxidase staining method using an Elite Vectastain ABC kit (Vector Laboratories Inc., Burlingame, CA). Specimens were stained for CD4$^+$ and CD8$^+$ cells with biotin-conjugated rat anti-mouse CD4 mAb (clone YTS 191.1.2, Cedarlane Laboratories Ltd., Hornby, Ontario, Canada) and biotin-conjugated rat anti-mouse CD8 mAb (clone 53-6.7, Pharmingen, Franklin Lakes, NJ), respectively. Intragraft monocyte/macrophage infiltration was detected by staining with biotin-conjugated rat anti-mouse Mac-1 mAb (Cedarlane Laboratories Ltd., Hornby, Ontario, Canada). Mouse IgG and IgM deposition in grafts was detected using biotin-conjugated goat anti-mouse-IgG and goat anti-mouse-IgM (Cedarlane). For identification of complement deposition, sections were serially incubated with goat anti-C3 or anti-C5 polyclonal Abs (Quidel, San Diego, CA), biotinylated rabbit anti-goat IgG (Vector Laboratories), and HRP-conjugated-streptavidin (Zymed Laboratories, South San Francisco, CA). Slides were washed with phosphate-buffered saline between steps, and examined under light microscopy. Negative controls were performed by omitting the primary antibodies. The immunostaining was scored in five high-power fields of each section, and five independent experiments were performed. The sections of immunoperoxidase staining were graded from 0 to 4+ according to the staining intensity: 0, negative; 1+, equivocal; 2+, weak staining; 3+, moderate staining; and 4+, very intensive staining.

*Flow Cytometry* The circulating anti-donor specific IgG and IgM antibodies were evaluated in the recipient serum by FACScan flow cytometry (Becton Dickinson, Mountain View, CA). Glotz et al., (1993); Tyan et al. (1994). Briefly, C3H mouse splenocytes were isolated and incubated at 37°C for 30 minutes with serum from naive control and experimental groups. To stain for total IgG, IgG1, IgG2a, IgG2b and IgM, the cells were washed and incubated with FITC-conjugated goat antibody specific for the Fc portion of mouse IgG or with phycoerythrin-conjugated goat antibody specific for the μ-chain of mouse IgM (Jackson ImmunoResearch Laboratories, West Grove, PA), or with FITC-conjugated goat anti-mouse IgG1 (CALTAG Laboratories, Burlingame, CA), or with FITC-conjugated goat anti-mouse IgG2a (CALTAG), or with FITC-conjugated goat anti-mouse IgG2b (CALTAG). After 1 hour of staining at 4°C, the cells were washed with PBS, resuspended at $5 \times 10^6$/mL, and analyzed by flow cytometry for mean channel fluorescence intensity, which represents the antibody-binding reactivity.

*Complement Hemolytic Assay* The purified anti-C5 mAb was serially diluted twofold (175-0.1 μg/ml) in GVB$^{2+}$ buffer (gelatin Veronal-buffered saline: 0.1% gelatin, 141 mM NaCl, 0.5 mM MgCl$_2$, 0.15 mM CaCl$_2$, and 1.8 mM sodium barbital) and added in triplicate (50 μl/well) to a 96-well plate. BALB/c mouse serum was diluted to 40% v/v with GVB$^{2+}$ buffer and added (50 μl/ml) to the rows of the same 96-well plate such that the final concentration of BALB/c mouse serum in each well was 20%. The plate was then incubated at room temperature for approximately 30 min while chicken erythrocytes were prepared. Chicken erythrocytes were washed 5×1 ml with GVB$^{2+}$ buffer and resuspended to a final concentration of 5×10$^7$/ml in GVB$^{2+}$. Four milliliters of the chicken erythrocytes were sensitized by adding anti-chicken RBC polyclonal antibody (Intercell Technologies, Hopewell, NJ, 0.1 % v/v) and the cells were incubated at 4 °C for 15 min with frequent vortexing. The cells were then washed 2×1 ml with GVB$^{2+}$ and resuspended to a final volume of 2.4 ml in GVB$^{2+}$. The chicken erythrocytes (30 μl/well, 2.5×10$^6$ cells) were added to the plate containing serum and anti-C5 mAb as described above, mixed well, and incubated at 37 °C for 30 min. The plate was then centrifuged at 1000×g for 2 min, and 85 μl of the supernatant was transferred to a new 96-well microtiter plate. The plate was read at OD 415 nm using a microplate reader and the percentage of hemolysis was determined using this formula:

$$\% \text{ hemolysis} = 100 \times \frac{(OD \text{ sample}) - (OD \text{ GVB}^{2+} \text{ control})}{(OD \text{ 100\% lysed control}) - (OD \text{ GVB}^{2+} \text{ control})}$$

with 100% lysed control obtained by the addition of 100 μl GVB$^{2+}$ containing 0.1% NP-40 to the 30 μg/ml of chicken erythrocytes as prepared above.

*Western Blot Analysis* Sonication of frozen heart samples was performed in RIPA lysis buffer (Santa Cruz Biotechnology, Inc.) at 4 °C for 1 minute at 10-second intervals, followed by microcentrifugation at 13,000 rpm for 10 minutes at 4 °C. Clarified supernatants were immediately quantitated in triplicate for protein content using Detergent-compatible protein assay kit (BIO-RAD). Heart lysates (10μg protein/well) were separated on NuPAGE, 4-12% gradient Bis-Tris gels and MES buffer system (Invitrogen) and transferred to polyvinylidene difluoride (PVDF) membrane (0.45 μm pore size; Invitrogen) using a semi-dry transfer apparatus (BIO-RAD). Membranes were cut appropriately at the correct molecular weights to allow the development of the blots with two different primary antibodies per blot such that each blot was exposed to a test antibody and an internal control antibody to insure equal sample loading. The test primary antibodies including anti-Bcl-2 (N-19) rabbit polyclonal sera (Santa Cruz Biotechnology, Inc.) and anti-Bcl-XS/L (M-125) rabbit polyclonal sera (Santa Cruz Biotechnology, Inc.) were used to detect intragraft expression of Bcl-2 and Bcl-xl proteins. Anti-calsequestrin rabbit polyclonal sera (Calbiochem) were used as internal control primary antibody (Kobayashi et al., 1999). Detection of primary antibody binding was performed as previously described (Arp et al., 1996) by exposing washed incubated blots to a polyclonal goat anti-rabbit IgG fraction conjugated to horseradish peroxidase (HRP; Roche Laboratories) and then appropriately developed by exposure to enhance chemiluminescence for HRP-conjugated antibodies (Roche Laboratories).

*Statistical Analysis* The data were reported as the mean ± SD. Allograft survival among experimental groups was compared using the rank-log test. Histological and immunohistological findings were analyzed using the Mann-Whitney U test. Flow cytometric data and western blot data were analyzed using one-way ANOVA. Differences with p values less than 0.05 were considered significant.

Example 2

*Presensitization with C3H donor skin graft induces antibody-mediated ACHR in heart allografts of BALB/c recipients.*

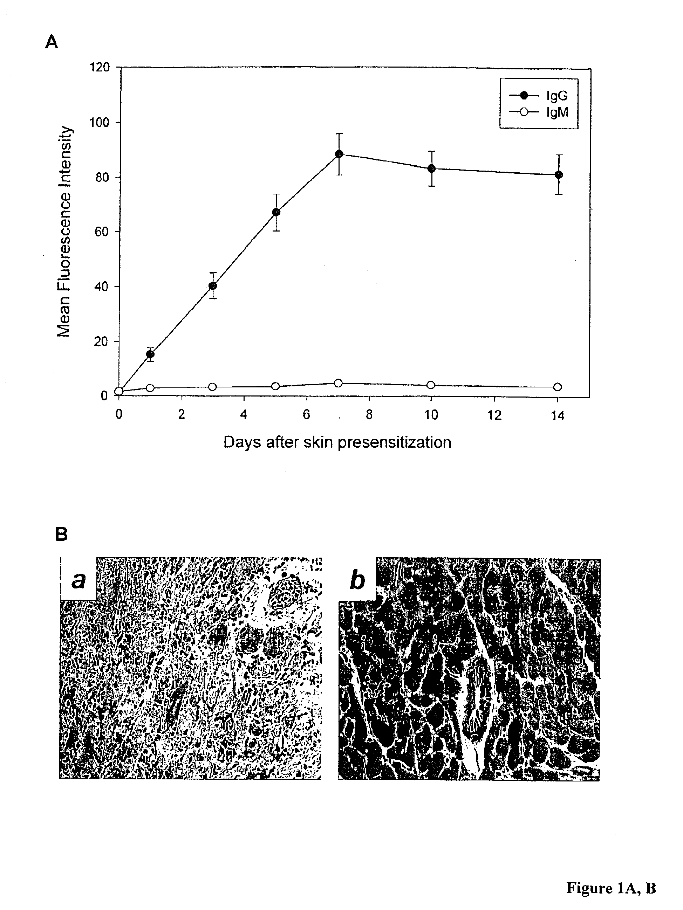

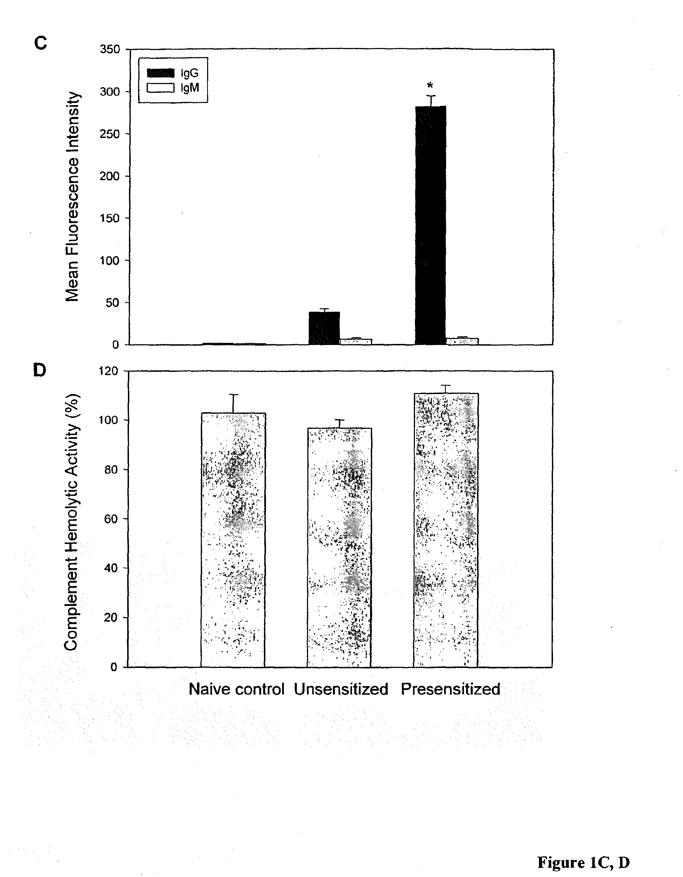

To develop a suitable small animal model that mimics presensitized patients in the clinic and to study ABMR, a novel, fully MHC-mismatched mouse ABMR model has been developed through presensitization of mouse recipients. In this model, BALB/c recipients were presensitized with C3H donor skin grafts one week prior to heart transplantation from the same donor. Seven days after donor skin presensitization, serum level of anti-donor IgG, but not IgM antibody was markedly elevated and reached to a peak level in the presensitized BALB/c recipients (Figure 1A). Heart transplantation from same donor was then performed in these highly sensitized recipients. Without immunosuppression, C3H heart grafts were rapidly rejected in 3.1 ± 0.4 days by ACHR, characterized by severe thrombosis, hemorrhage and infarction (Figure 1B-a). In contrast, same heart grafts in unsensitized BALB/c recipients (with mean survival time, MST of 8.2 ± 0.8 days) show the normal histology on post-operative day (POD) 3 (Figure 1B-b). When compared to unsensitized BALB/c recipients at the same day, heart grafts in presensitized animals revealed massive IgG antibody and complement (C3 and C5) deposition, but minimal CD4$^+$ and CD8$^+$ cell infiltration (Table 1). Furthermore, circulating anti-donor IgG levels in presensitized recipients were significantly higher than those of unsensitized same recipients receiving a heart graft on POD3 (P< 0.01, Figure 1C). However, anti-donor IgM remained at very low levels both in circulation (Figure 1C) and in heart grafts (Table 1) and it showed no significant difference between unsensitized and presensitized recipients. In addition, normal levels of complement hemolytic activity were shown in both presensitized and unsensitized heart recipients without treatment (Figure 1D). These data indicate that this is an ideal transplant model to study ABMR in presensitized recipients in which complement plays an important role in the pathogenesis.

Table 1.

Comparison of immunohistological changes of C3H heart allografts in unsensitized and presensitized BALB/c recipients on POD3

| Groups | Unsensitized | Presensitized |
|---|---|---|
| IgG | 1+ | 4+ |
| IgM | 1+ | 1+ |
| C3 | 2+ | 3+ |
| C5 | 2+ | 3+ |
| CD4 | 0 | 1+ |
| CD8 | 0 | 1+ |

Grades for immunoperoxidase staining: 0, negative; 1+, equivocal; 2+, weak;
3+, moderate; 4+, intense.

*Anti-C5 mAb in combination with CsA and CyP prevents ABMR and achieves indefinite heart allograft survival in presensitized mouse recipients.*

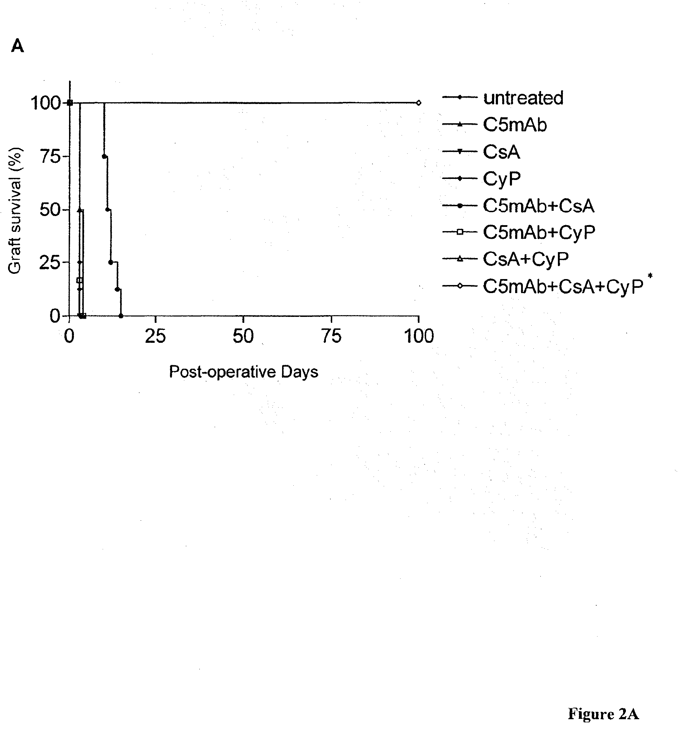

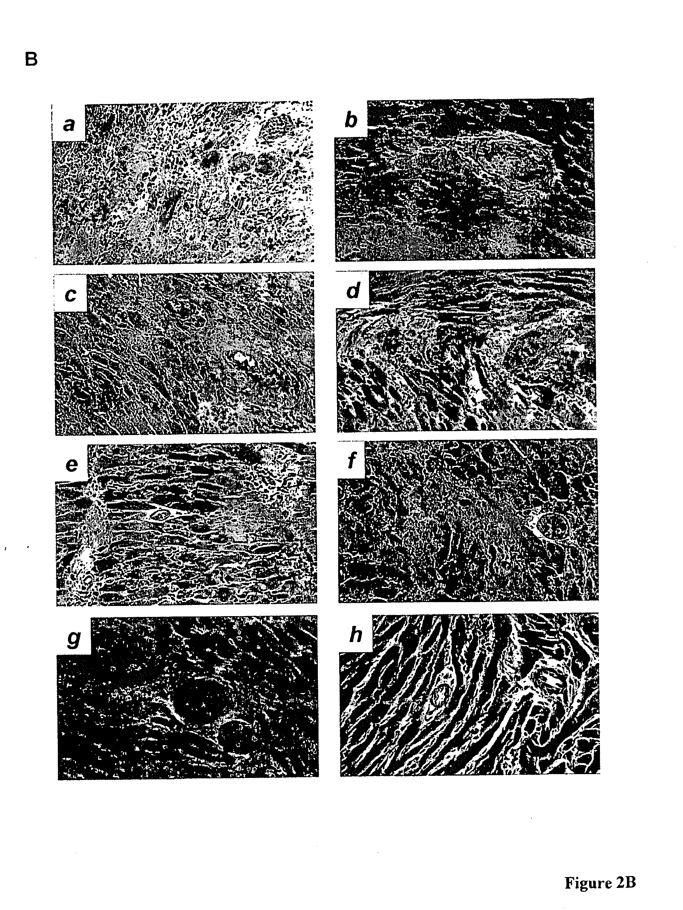

Complement has been shown to play an important role in ABMR. However, the inhibitory effect of functionally blocking terminal complement cascade at the C5 level in highly sensitized recipients is unknown. In the study presented herein, the presensitized model was used to study the efficacy of anti-C5 mAb either alone or combined with CsA and /or CyP in prevention of ABMR. As presented in Figure 2A, treatment with either CsA or CyP or the two drugs in combination did not prevent ABMR and grafts were rejected in $3.0 \pm 0.0$ days, $3.3 \pm 0.5$ days and $3.5 \pm 0.6$ days, respectively with typical pathological features of ACHR including intravascular thrombosis and interstitial hemorrhage (Figure 2B-b, c, d), which were indistinguishable from heart grafts in untreated presensitized BALB/c recipients (Figure 2B-a). Anti-C5 monotherapy or combined with CyP was not able to improve graft survival and heart grafts were rejected by ACHR (Figure 2B-e, f) in $3.5 \pm 0.6$ days and $3.2 \pm 0.4$ days, respectively (Figure 2A). Although the combination therapy of anti-C5 mAb and CsA, the protocol capable of inducing long-term heart allograft survival in unsensitized animals, marginally prolonged graft survival in this presensitized model, heart grafts were also rejected by severe humoral rejection with vasculitis, thrombosis, hemorrhage and minimal cell infiltration (Figure 2B-g) in $11.9 \pm 1.8$ days (Figure 2A). In contrast, triple therapy of anti-C5 mAb in combination of CsA and CyP achieved indefinite heart graft survival over 100 days (Figure 2A) in presensitized animals (P<0.01 vs. the animals without treatment or treated with either monotherapy or two drugs in combination) with no evidence of rejection (Figure 2B-h). In this presensitized mouse model, as shown in Table 2, only minor intragraft CD4$^+$ and CD8$^+$ cell infiltration was observed in the recipients that rejected their heart grafts within 3 days. However, the number of these T cells was slightly increased if heart grafts survived longer in anti-C5 mAb plus CsA-treated recipients at the time of rejection (POD11) and in triple therapy-treated recipients at early stages of graft survival (e.g. POD11). Furthermore, with continuous treatment of CsA in the triple therapy group, CD4$^+$ and CD8$^+$ cell infiltration was inhibited in long-term surviving heart grafts on POD60 and 100. In addition, moderate intragraft Mac-1$^+$ cell infiltration, including monocytes and macrophages, was found in untreated and CsA-, CyP- or CsA plus CyP-treated animals, while the infiltration of these cells was significantly reduced in anti-C5 mAb treated animals (Table 2). These results indicate that functionally blocking anti-C5 mAb enables the use and efficacy of conventional immunosuppressive agents, thereby preventing ABMR and achieving indefinite heart graft survival in presensitized recipients.

Table 2.

Grades for immunoperoxidase staining of heart allografts in presensitized mouse recipients at necropsy

| Groups | Date for sample collection (POD) | C3 | C5 | **CD4** | CD8 | Mac-1 | IgG | IgM |
|---|---|---|---|---|---|---|---|---|
| Untreated | 3 | 3+ | 3+ | 1+ | 1+ | 3+ | 4+ | 1+ |
| CsA | 3 | 3+ | 3+ | 1+ | 1+ | 3+ | 4+ | 1+ |
| CyP | 3 | 3+ | 3+ | 1+ | 1+ | 3+ | 3+ | 1+ |
| CsA+CyP | 3 | 3+ | 3+ | 1+ | 1+ | 3+ | 3+ | 1+ |
| Anti-C5mAb | 3 | 3+ | 0 | 1+ | 1+ | 2+ | 4+ | 1+ |

(continued)

| Grades for immunoperoxidase staining of heart allografts in presensitized mouse recipients at necropsy | | | | | | | | |
|---|---|---|---|---|---|---|---|---|
| *Groups* | Date for sample collection (POD) | C3 | C5 | **CD4** | CD8 | Mac-1 | IgG | IgM |
| Anti-C5mAb+CsA | 11 | 3+ | 0 | 2+ | 2+ | 2+ | 4+ | 1+ |
| Anti-C5mAb+CyP | 3 | 3+ | 0 | 1+ | 1+ | 2+ | 3+ | 1+ |
| Anti-C5mAb+CsA+CyP | 3 | 3+ | 0 | 1+ | 1+ | 2+ | 3+ | 1+ |
| Anti-C5mAb+CsA+CyP | 11 | 3+ | 0 | 2+ | 2+ | 1+ | 3+ | 1+ |
| Anti-C5mAb+CsA+CyP | 60 | 3+ | 0 | 1+ | 1+ | 0 | 2+ | 1+ |
| Anti-C5mAb+CsA+CyP | 100 | 3+ | 2+ | 0 | 0 | 0 | 2+ | 1+ |

Grades: 0, negative; 1+, equivocal; 2+, weak; 3+, moderate; 4+, intense.

*Anti-C5 mAb completely inhibits total complement hemolytic activity and local C5 deposition in presensitized recipients receiving a heart allograft.*

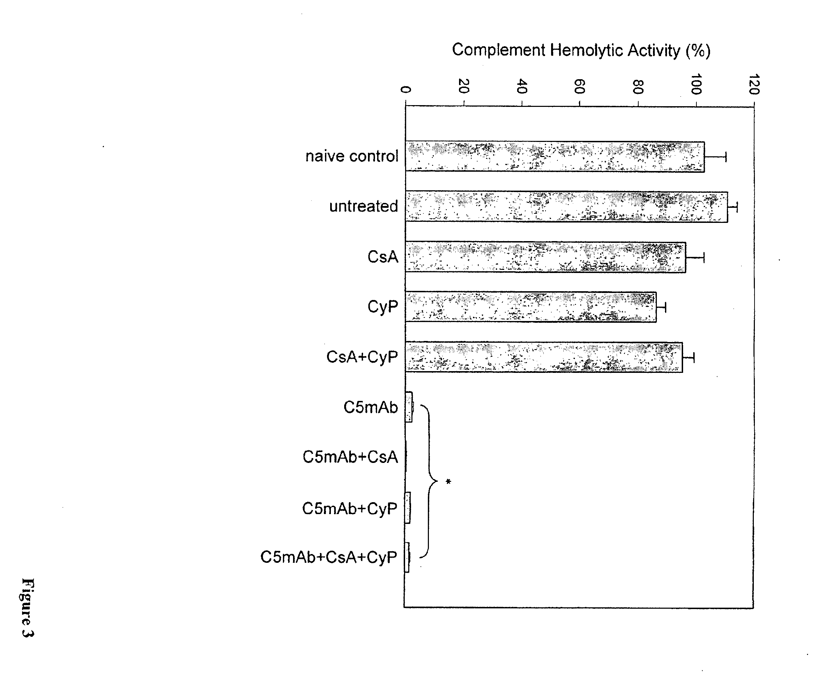

Anti-C5 mAb was previously shown to block the cleavage of complement protein C5 into the proinflammatory molecules C5a and C5b-9 (Kroshus et al., 1995), and to completely and consistently block terminal complement activity in mice (Wang et al., 1999). In the current study, terminal complement activity was measured by assessing the ability of recipient mouse sera to lyse antibody presensitized chicken erythrocytes and was compared at the same time-point (POD3). Treatment of mice with either CsA or CyP or the two drugs in combination had no effect on terminal complement activity, while treatment with anti-C5 mAb either alone or combined with CsA or/and CyP completely inhibited this activity (Figure 3; P<0.01, vs. naive and untreated animals, as well as CsA-, CyP-, or CsA plus CyP-treated animals). In addition, sera obtained from anti-C5 mAb treated animals at several earlier time points showed similarly diminished hemolytic activity, suggesting that serum terminal complement was inhibited throughout the treatment period. Furthermore, local C5 deposition in heart grafts was completely prevented in the anti-C5 mAb treated presensitized recipients, but not in untreated, or CsA-, CyP- and CsA plus CyP-treated presensitized animals (Table 2). As predicted, treatment with anti-C5 mAb did not prevent C3 deposition in the grafts (Table 2). These results suggest anti-C5 therapy completely blocks total complement activity after cardiac allografting in highly sensitized recipients.

*Long-term surviving heart grafts in presensitized animals are resistant to humoral injury in the presence of low level of anti-donor antibodies and complement- a situation of accommodation.*

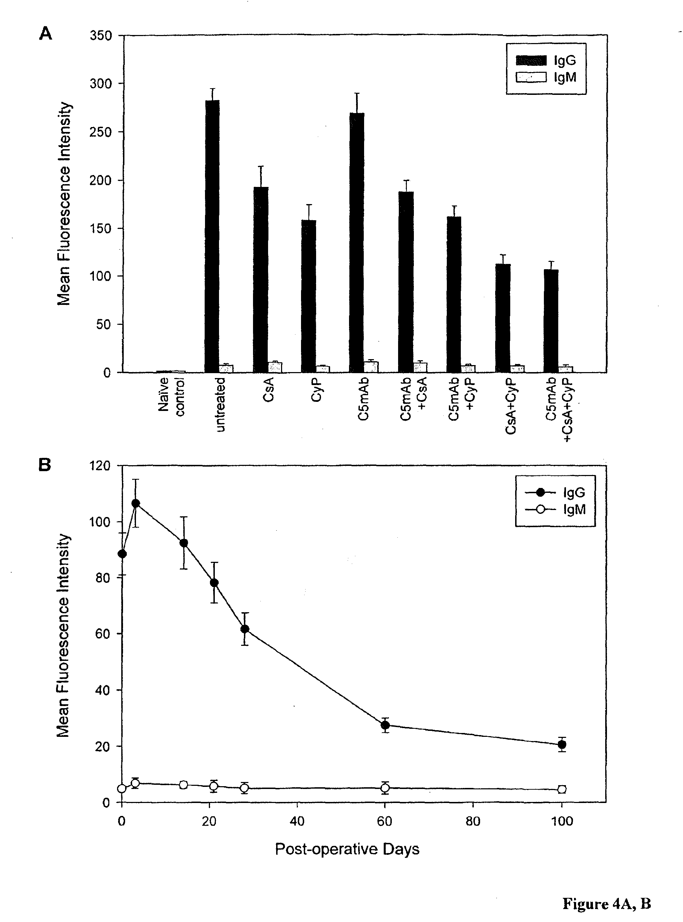

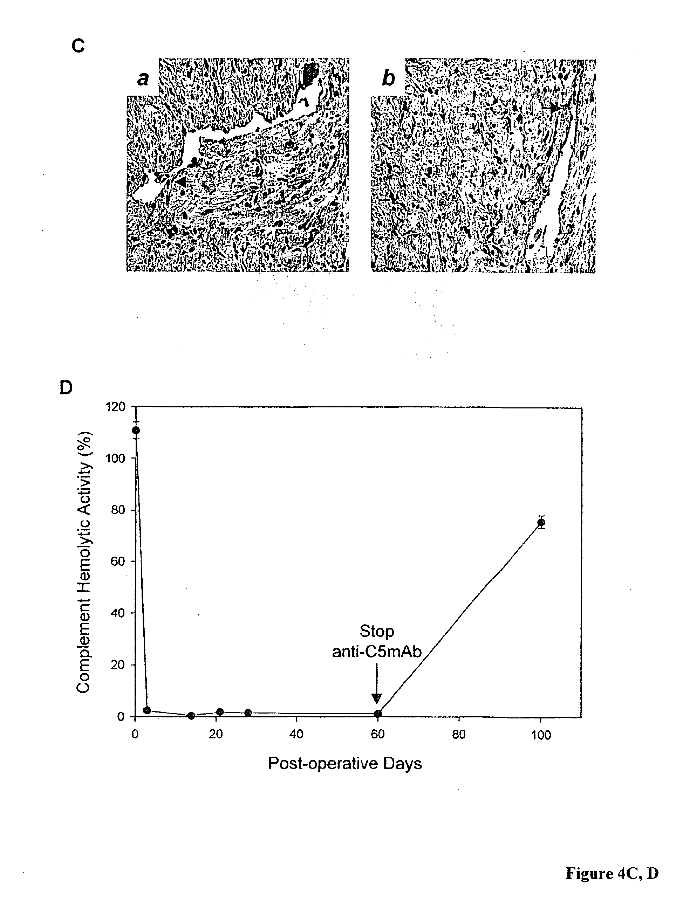

To further investigate the role of anti-C5 mAb in humoral rejection, anti-donor alloantibody levels were measured in recipient sera by flow cytometry and intragraft antibody deposition by using immunostaining techniques in different groups. Figure 4A shows that on POD3 untreated presensitized BALB/c recipients had high levels of circulating anti-donor IgG antibodies. When presensitized recipients receiving either monotherapy or two drugs in combination, CsA and/or CyP partially down-regulated circulating anti-donor IgG levels, while treatment with anti-C5 mAb either alone or combined with CsA or CyP did not further affect anti-donor antibody levels at the same day. In contrast, with triple therapy of anti-C5 mAb, CsA and CyP, a high level of circulating anti-donor IgG was gradually downregulated and reached a low level on POD60, thereafter remaining at this level until day 100 (Figure 4B). Similar to levels of circulating antibodies in the different treatment groups, Table 2 shows that strong deposition of anti-mouse IgG was present in the rapidly rejected heart grafts of presensitized animals with no treatment or treated with monotherapy or two drugs in combination therapy. Interestingly, with triple therapy, IgG antibody deposition was gradually attenuated to a mild level in the long-term surviving heart grafts on POD 100 (Figure 4C-a, Table 2). In this model, IgM remained at very low levels in either circulation (Figure 4A. B) or transplanted heart grafts (Figure 4C-b, Table 2) in presensitized recipients with or without treatment. In addition, treatment with anti-C5 mAb eliminated complement activity to an undetectable level until day 60, followed by a progressive recovery to predepletion levels on POD 100 after discontinuation of anti-C5 therapy in presensitized mouse recipients receiving triple therapy (Figure 4D). Furthermore, intragraft C5 deposition was also detected in 100-day surviving presensitized animals (Table 2). These data demonstrate that ongoing transplant accommodation occurs in triple therapy treated presensitized recipients despite the presence of anti-graft antibodies and complement activation.

*Anti-C5 mAb in combination with CsA and CyP reduces the IgG1/IgG2α ratio and leads to a shift in IgG subclass to IgG2b in recipients with accommodated grafts.*

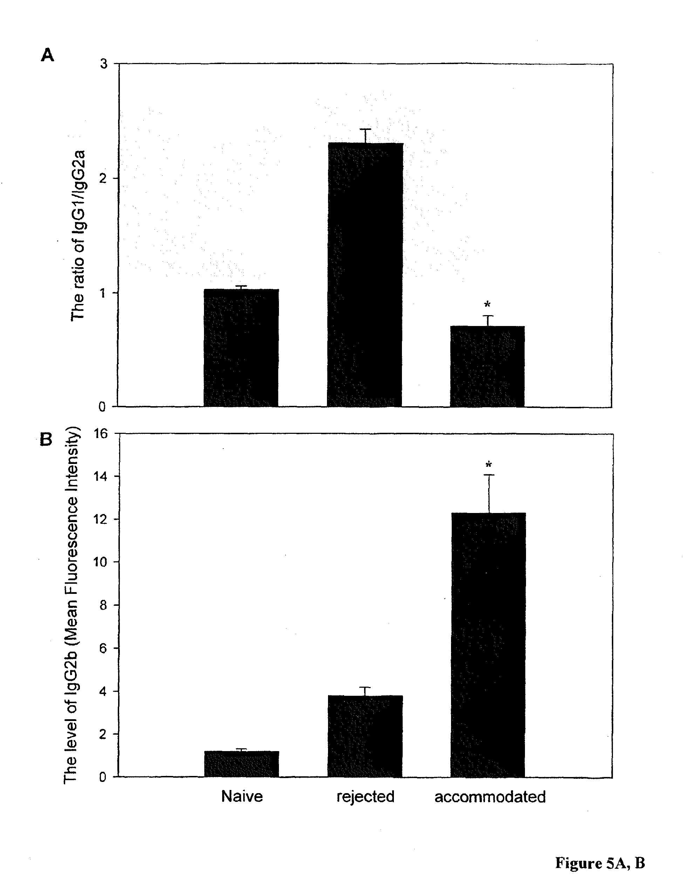

To determine whether anti-C5 mAb-based triple therapy would induce a shift in IgG subclass, which may be associated with accommodation, serum levels of anti-donor IgG subclasses of IgG1, IgG2a and IgG2b were compared between untreated recipients and the recipients with accommodated heart graft. Sera from untreated recipients contained predominant IgG1 isotype, indicated by a high ratio of IgG1/IgG2a (Figure 5A). In contrast, a significant reduction in the ratio of IgG1/IgG2a was observed in the recipients carrying accommodated grafts (Figure 5A, P<0.01). Furthermore, presensitized recipients with the accommodated heart grafts displayed an increased level of anti-donor IgG2b as compared to the same recipients with rejected grafts (Figure 5B, P<0.01). In addition, the pattern of IgG isotypes in the recipients treated with either monotherapy or two drugs in combination is indistinguishable from that of untreated animals. These data indicate that anti-donor IgG1 isotype may be associated with graft rejection, while production of anti-donor IgG2b subclass may function as a protective antibody and plays an important role in the induction of accommodation.

*Anti-C5 mAb in combination with CsA and CyP induces intragraft Bcl-2 and Bcl-xl expression in highly sensitized mouse recipients.*

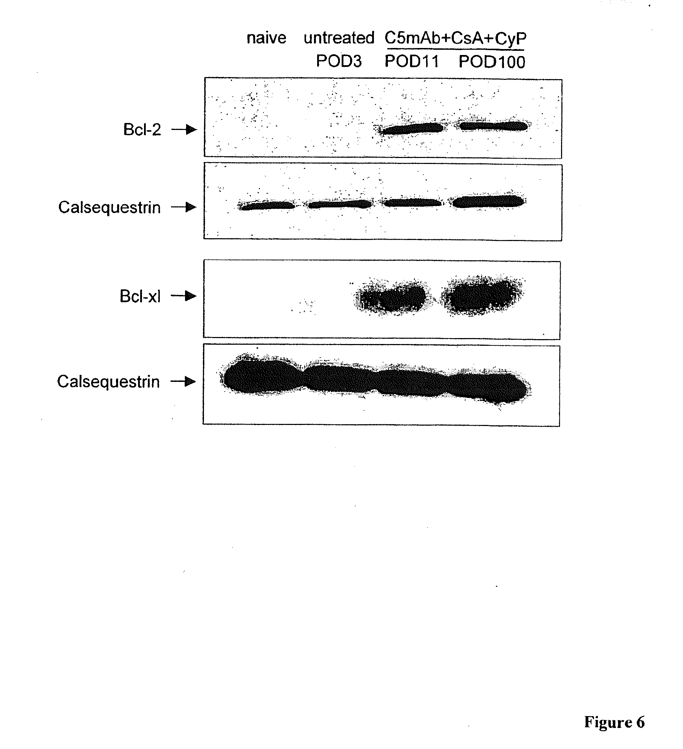

To determine whether a causal relationship exists between intragraft expression of protective proteins and graft resistance to humoral injury in this model, western blot analysis was employed to detect proteins of interest in heart graft tissues from highly sensitized mouse recipients. Long-term surviving heart grafts were found to express high levels of Bcl-2 and Bcl-xl proteins on POD 100, and these proteins were detected as early as 12 days after heart transplantation in highly sensitized recipients receiving anti-C5 mAb-based triple therapy (Figure 6). In contrast, there were no Bcl-2 and Bcl-xl proteins expressed on heart grafts of untreated animals (Figure 6) or animals treated with either monotherapy or two drugs in combination therapy. This result suggests that graft resistance to humoral injury in indefinite surviving animals is associated with the protection provided by Bcl-2 and Bcl-xl proteins in this presensitized model.

*Presensitized recipients with an accommodating first heart graft accept a second accommodated heart graft but reject a second naive heart graft from the same donors.*

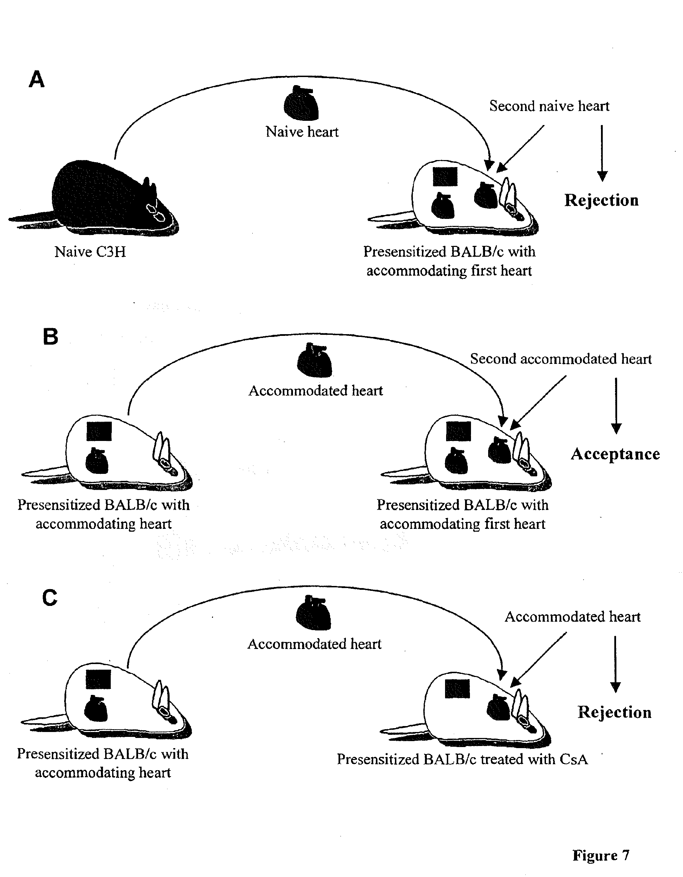

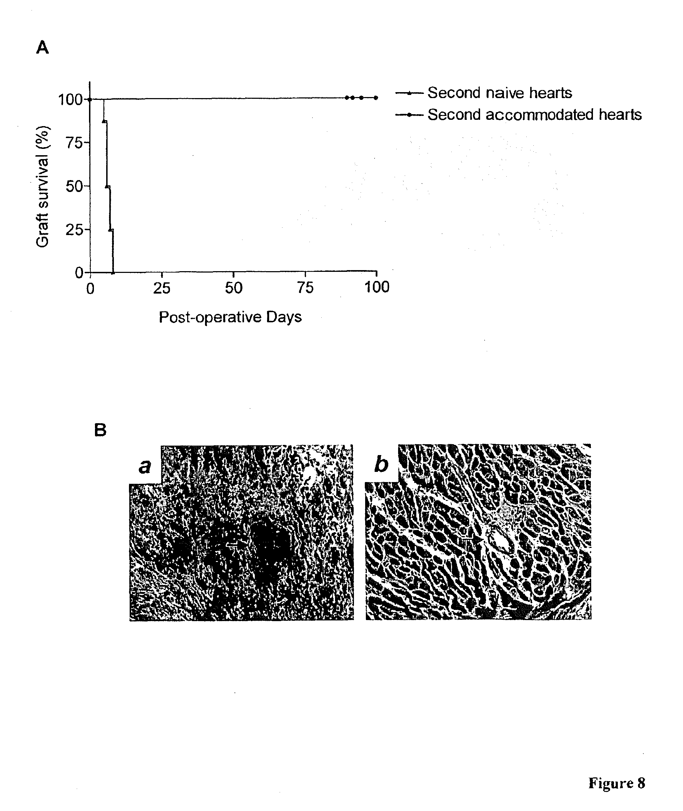

The ability of accommodated grafts to resist rejection has not been tested directly under pathophysiological conditions where naive grafts undergo rejection following allotransplantation. In this model, to determine whether presensitized recipients with an accommodating first heart graft will accept a second accommodated graft but reject a second naive graft, we performed re-transplantation scenarios. After the accommodated C3H heart grafts have survived to the 100-day point; the time at which low levels of alloantibodies were detected (Figure 4B) and complement activity has returned to pretreatment levels (Figure 4D), in presensitized BALB/c recipient treated with anti-C5 mAb-based triple therapy, these recipients received a second heart graft. Specifically, either a naive (Figure 7A) or a 100-day accommodated C3H heart (Figure 7B) from another presensitized BALB/c recipient was transplanted into the neck of the presensitized recipients carrying an accommodating first C3H heart. These recipients rejected a second naive heart at 6.6 ± 1.1 days (Figure 8A) with severe AVR (Figure 8B-a) while the first heart continued to survive. In contrast, when the accommodated hearts that had been already surviving for 100 days in different presensitized mice were used as second grafts, these grafts were accepted by the presensitized recipients carrying an accommodating first heart graft (Figure 8A). There was no sign of rejection in those accommodated second heart grafts 90 days after second transplantation (Figure 8B-b). These data indicate that accommodated grafts become resistant to the effects of anti-donor antibodies and complement that normally mediate allograft rejection in these presensitized recipients. Furthermore, the fact that the host of the accommodated graft rejected a new graft suggests that accommodation involves changes to the graft.

*Presensitized recipients being treated with CsA reject accommodated heart grafts.*

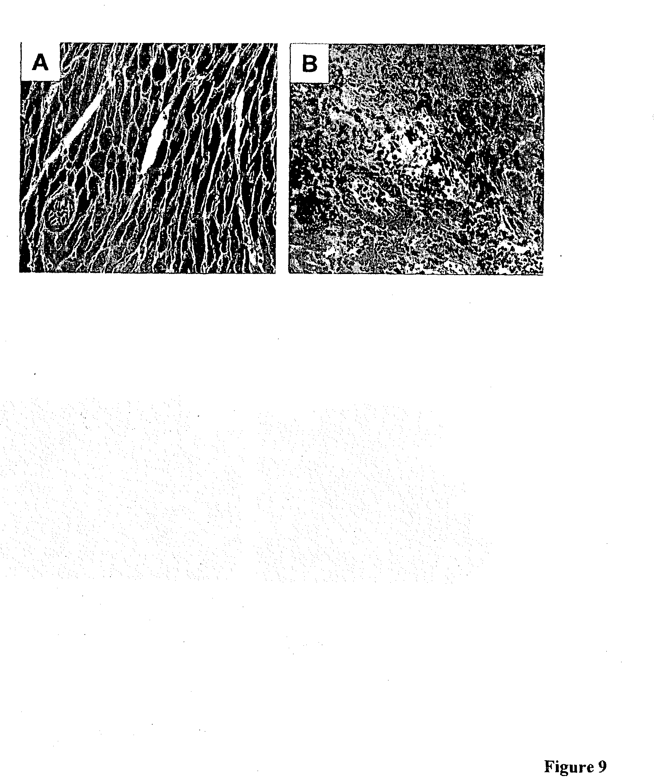

Another re-transplantation was performed to determine whether accommodation in this presensitized model would be caused by the changes in the grafts and/or the recipients. Specifically, after C3H heart grafts have been accommodated in presensitized BALB/c mice for 100 days, the accommodated heart graft will then be re-transplanted into a second presensitized BALB/c recipient being treated with CsA alone (Figure 7C), a therapy that can prevent cellular rejection but cannot prevent accelerated humoral rejection of a fresh C3H heart in presensitized recipients. The accommodated C3H heart grafts were rapidly rejected in CsA treated presensitized BALB/c recipients. After re-transplantation, the pathology in accommodated heart grafts was changed from normal (Figure 9A) to severe ACHR with massive interstitial hemorrhage but few cell infiltrates (Figure 9B). In addition, high levels of anti-donor IgG and normal levels of complement hemolytic activity in these recipients receiving an accommodated C3H heart were similar to those of CsA treated presensitized recipients receiving a naive C3H heart. This result further indicates that accommodation induced by anti-C5 mAb-based triple therapy can originate from mechanisms involving changes not only to the graft, but also to the recipient.

<u>Example 3</u>

*Acute Vascular Rejection in a Heart Transplantation Model*

Experiments were performed to determine whether inclusion of an inhibitor of formation of terminal complement would attenuate acute vascular rejection and whether the use of such an inhibitor in conjunction with an immunosuppressant would achieve long-term allograft survival. In this set of experiments an anti-C5 monoclonal antibody was used in conjunction with cyclosporin. The model used was an allograft heterotopic heart transplant from C3H mice into BALB/c mice. This model is a stringent acute vascular rejection model with the C3H and BALB/c mice being strongly MHC mismatched. The transplantations and other methods were performed as described in Wang et al. (2003).

*Heterotopic Cardiac Transplantation*

Intra-abdominal heterotopic cardiac transplantation was performed as previously described by Wang et al. (2003). Briefly, a median sternotomy was performed on the donor, and the heart graft was slowly perfused in situ with 1.0 ml of cold heparinized Ringer's lactate solution through the inferior vena cava and aorta before the superior vena cava and pulmonary veins were ligated and divided. The ascending aorta and pulmonary artery were transected, and the graft was removed from the donor. The graft was then revascularized with end-to-side anastomoses between the donor's pulmonary artery and the recipient's inferior vena cava as well as the donor's aorta and the recipient's abdominal aorta using 11-0 nylon suture. The beating of the grafted heart was monitored daily by direct abdominal palpation. The degree of pulsation was scored as: A, beating strongly; B, noticeable decline in the intensity of pulsation; or C, complete cessation of cardiac impulses. When cardiac impulses were no longer palpable, the graft was removed for routine histology. In certain instances, mice in which the graft was still functioning were sacrificed to perform histology.

*Results*

Mice (male 8-12 week old mice weighing 25 - 30 g) were split into six experimental groups with six to eight mice per group. Transplant occurred on day 0. Histological changes were checked at the endpoint (the endpoint being graft failure) or in some cases a mouse was sacrificed prior to graft failure. The dosage of BB5.1 which was administered (40 mg/kg body weight three times per week) was known from prior studies to completely inhibit terminal complement activity.

Group 1' (control) - mice were administered 0.75 mL of saline intraperitoneally on days -1, 0, 1 and 2. Subsequently these mice were treated with 0.75 mL of saline intraperitoneally three times per week (Monday, Wednesday, Friday) until the endpoint.

Group 2' (cyclosporin A alone) - mice were administered 15 mg/kg body weight of cyclosporin A subcutaneously on a daily basis beginning at day 0 (day of transplant) until the endpoint.

Group 3' (anti-complement antibody alone) - mice were administered the anti-mouse C5 antibody BB5.1 (Frei et al., 1987) at 40 mg/kg body weight intraperitoneally on days -1, 0, 1 and 2 followed by 40 mg/kg body weight administered three times per week (Monday, Wednesday, Friday) until the endpoint.

Group 4' (anti-complement antibody until day 14 post-transplant plus cyclosporin A) - mice were administered the anti-mouse C5 antibody BB5.1 at 40 mg/kg body weight intravenously on days -1 through day 14 and were also administered cyclosporin A at 15 mg/kg of body weight on a daily basis beginning at day 0 until the endpoint. Note that this differs from the other groups in that the BB5.1 was administered intravenously and on a daily basis.

Group 5' (anti-complement antibody until day 28 post-transplant plus cyclosporin A) - mice were administered the anti-mouse C5 antibody BB5.1 at 40 mg/kg body weight intraperitoneally on days -1,0,1 and 2 followed by 40 mg/kg body weight administered three times per week (Monday, Wednesday, Friday) until day 28 and were also administered cyclosporin A at 15 mg/kg of body weight on a daily basis beginning at day 0 until the endpoint.

Group 6' (anti-complement antibody chronically until 100 days plus cyclosporin) - mice were administered the anti-mouse C5 antibody BB5.1 at 40 mg/kg body weight intraperitoneally on days -1,0, 1 and 2 followed by 40 mg/kg body weight administered three times per week (Monday, Wednesday, Friday) until 100 days and were also administered cyclosporin A at 15 mg/kg of body weight on a daily basis beginning at day 0 until 100 days.

The results of this experiment are shown in Tables 3 and 4. Table 3 shows the survival time for the grafts. Table 4 sets forth the histological scores.

Table 3

| Allograft Survival | | |
|---|---|---|
| Group (Treatment) | Individual Survival (days) | Mean Survival Time (days) |
| 1'. Saline | 8, 8, 8, 8, 8, 9 | 8.3 ± 0.5 |
| 2'. Cyclosporin A | 14, 15, 15, 16, 16, 16, 17 | 15.5 ± 1.1 |
| 3'. BB5.1 | 7, 8, 8, 8, 8, 9 | 8.0 ± 0.6 |
| 4'. BB5.1 until day 14 + cyclosporin A | 35, 38, 43, 45, 46, 47 | 42.3 ± 4.8 |
| 5'. BB5.1 until day 28 + cyclosporin A | 77, 80, 80, 81, 82 | 80 ±1.9 |
| 6'. BB5.1 until day 100 + cyclosporin A | >100 days (7 mice; one sacrificed for histology) | > 100 days |

Table 4

| Median Scores of Histological Changes of Heart Allografts at Necropsy | | | | | | | |
|---|---|---|---|---|---|---|---|
| Groups | Vasc* | Infarc | Lymph | Throm | Hemo | Fibrin | PMN |
| 1'. Saline (endpoint) | 3.0 | 3.0 | 1.0 | 4.0 | 3.0 | 3.0 | 3.0 |
| 2'. Cyclosporin A (endpoint) | 2.0 | 1.0 | 2.0 | 3.0 | 2.0 | 2.0 | 3.0 |
| 3'. BB5.1 (endpoint) | 2.0 | 1.0 | 2.0 | 2.0 | 1.0 | 0.0 | 0.0 |
| 4'. BB5.1 until day 14 + cyclosporin A | N/A | N/A | N/A | N/A | N/A | N/A | N/A |
| 5'. BB5.1 28 days + Cyclosporin A (post-operative day 8) | 0.0 | 0.0 | 0.0 | 0.0 | 0.0 | 0.0 | 0.0 |
| 5'. BB5.1 28 days + Cyclosporin A (endpoint) | 0.0 | 0.0 | 1.0 | 1.0 | 2.0 | 1.0 | 0.0 |
| 6'. BB5.1 until day 100 + Cyclosporin A (post-operative day 100) | 0.0 | 0.0 | 0.0 | 0.0 | 0.0 | 0.0 | 0.0 |
| Median scores: 0 - normal; 1- minimum change; 2 - mild change; 3 - moderate change; 4 - marked change. N/A - not available. *Vasc - vasculitis; Infar - infarction; Lymph - lymphocyte infiltration; Throm - thrombosis; Hemo - hemorrhage; Fibrin - fibrin deposition; PMN - polymorphonuclear cell infiltrate | | | | | | | |

The results indicate the synergistic effects of using a complement inhibiting drug in addition to an immunosuppressant. In untreated mice the grafts were rejected in about 8 davs. Use of the immunosuppressant cyclosporin A alone on a daily, chronic basis resulted in an increase in graft survival until approximately 15 days post-transplant. The use of the anti-C5 antibody BB5.1 to inhibit formation of terminal complement had no effect on its own, graft rejection occurring at 8 days post-transplant as in the control group (Group 1'). The combination of BB5.1 through day 28 post-transplant plus cyclosporin A showed a synergistic effect with graft survival being extended until approximately day 80. A more surprising result is that of Group 5' in which BB5.1 and cyclosporin A were each administered chronically post-transplant. In this case the graft survival was for more than 100 days (as much data as presently available). Additionally, the histological results shown in Table 4 indicate that the administration of both BB5.1 and cyclosporin A protected the graft from changes much better than either BB5.1 or cyclosporin A alone, and that the chronic administration of BB5.1 and cyclosporin A protected the graft to such an extent that even at 100 days post-transplant there were no histological changes seen in the engrafted hearts. A survival time of 100 days in these models is considered to be the gold standard. A survival of 100 days in the model is believed to indicate that there will be an indefinite survival of the allograft. When BB5.1 administration was stopped after 28 days, the grafts were protected but they did begin to show some minimal to mild histological changes by about day 80 which was the time at which graft failure occurred.

The Group 4' mice were treated differently in that they were administered BB5.1 on a daily basis by an intravenous administration. These animals became ill, showing weight loss and urine retention and were sacrificed at a time at which the grafted hearts were still beating although their function had declined. This was the first group of mice studied and it is unknown why these ill effects were seen. These ill effects were not seen when the BB5.1 was administered intraperitoneally with a schedule of three times per week. As seen below in Example 4, daily administration of BB5.1 via an intraperitoneal route did not cause ill effects. Also, intravenous administration was not necessarily the cause of the illness in these animals. Intravenous administration of eculizumab (a human equivalent antibody to BB5.1 in that it binds to human C5) has been successfully administered intravenously without ill effects to humans in a study of PNH (Hillmen et al., 2004). Complement inhibitors may be administered by other routes in addition to intravenous and intraperitoneal, with all such routes being well known by those skilled in the art.

Example 4

*Accelerated Rejection in a Presensitized Heart Transplantation Model*

A second set of experiments similar to those of Example 3 was performed but the recipient mouse was presensitized to the donor organ. In these experiments, the presensitization was brought about by prior transplantation of a skin graft. In general, presensitization can occur not only as a result of having received an earlier allograft, but can also be caused by having received multiple blood transfusions or in women who have been pregnant. Besides such presensitization methods, allografts with an ABO mismatch will be rapidly attacked and rejected because of preformed antibodies to the ABO antigens unless steps are taken to prevent such an attack.

Some mice in these studies were administered cyclophosphamide in addition to BBS.1 and/or cyclosporin A. For these experiments BALB/c recipient mice were presensitized with C3H skin grafts one week prior to heart transplantation from the same donor (using the method of Pruitt and Bollinger, 1991). This model is designed to mimic presensitized transplantation in humans, especially in relation to accelerated humoral rejection. Recipient mice were split into eight groups of six to eight mice each. The treatments were as follow.

Group 1" (control) - mice (male 8-12 week old mice weighing 25 - 30 g) were administered 0.75 mL saline intraperitoneally on a daily basis beginning at day -1 and continuing until the endpoint (graft rejection).

Group 2" (cyclosporin A alone) - mice were administered cyclosporin A subcutaneously at a dose of 15 mg/kg body weight beginning on day 0 (day of transplant) until the endpoint.

Group 3" (BB5.1 alone) - mice were administered the anti-mouse complement monoclonal antibody BB5.1 at a dose of 40 mg/kg body weight delivered intraperitoneally on a daily basis beginning at day -1 and continuing until the endpoint.

Group 4" (cyclophosphamide alone) - mice were administered cyclophosphamide intravenously at a dose of 40 mg/kg body weight on each of days 0 and 1.

Group 5" (BB5.1 plus cyclosporin A) - mice were administered BB5.1 intraperitoneally at a dose of 40 mg/kg body weight on a daily basis beginning at day -1 and continuing until the endpoint. These mice were additionally administered cyclosporin A subcutaneously at a dose of 15 mg/kg body weight on a daily basis from day 0 until the endpoint.

Group 6" (BB5.1 plus cyclophosphamide) - mice were administered BB5.1 intraperitoneally at a dose of 40 mg/kg body weight on a daily basis beginning at day -1 and continuing until the endpoint. These mice were additionally administered cyclophosphamide intravenously at a dose of 40 mg/kg body weight on each of days 0 and 1.

Group 7" (cyclosporin A plus cyclophosphamide) - mice were administered cyclosporin A subcutaneously at a dose of 15 mg/kg body weight on a daily basis from day 0 until the endpoint. These mice were additionally administered cyclophosphamide intravenously at a dose of 40 mg/kg body weight on each of days 0 and 1.

Group 8" (BB5.1 plus cyclosporin A plus cyclophosphamide) - mice were administered BB5.1 intraperitoneally at a dose of 40 mg/kg body weight on a daily basis beginning at day -1 and continuing until 100 days. These mice were also administered cyclosporin A subcutaneously at a dose of 15 mg/kg body weight on a daily basis from day 0 until 100 days. These mice were additionally administered cyclophosphamide intravenously at a dose of 40 mg/kg body weight on each of days 0 and 1. Two mice in this group were sacrificed at day 60 for histological studies (no rejection had yet occurred) and the four remaining mice still had not rejected their grafts by day 100.

Additionally a control group of mice which was not presensitized and received only the saline treatment as for Group 1" was tested.

The results of these experiments are shown in Tables 5 and 6. Table 5 lists survival times for the grafts and Table 6 summarizes the histological results.

Table 5

| Allograft Survival | | |
|---|---|---|
| Groups (Treatment) | Individual survival (days) | Mean survival time (days) |
| No presensitization | 8, 8, 8, 8, 8, 9 | 8.3 ± 0.5* |
| 1". One skin presensitization | 3, 3, 3, 3, 3, 3, 3, 4 | 3.1 ± 0.4 |
| 2". Cyclosporin A | 3, 3, 3, 3 | 3.0 ± 0.0 |
| 3". BB5.1 | 3, 3, 4, 4 | 3.5 ± 0.6 |
| 4". Cyclophosphamide | 3, 3, 3, 4 | 3.3 ± 0.5 |
| 5". BB5.1 + Cyclosporin A | 10, 10, 11, 11, 12, 12, 14, 15 | 11.9 ± 1.8** |
| 6". BB5.1 + Cyclophosphamide | 3, 3, 3, 3, 3, 4 | 3.2 ± 0.4 |
| 7". Cyclosporin A + Cyclophosphamide | 3, 3, 3, 4, 4, 4 | 3.5 ± 0.6 |
| 8". BB5.1 + Cyclosporin A + Cyclophosphamide | > 100 days (4 mice) | > 100*** |
| *P < 0.01 group 1" vs. no presensitization<br>**P < 0.01 group 5" vs. groups 1"-4" and 6"-7".<br>***P<0.01 group 8" vs. groups 1"-7". | | |

Table 6

| Median Scores of Histological Changes of Heart Allografts at Necropsy | | | | | | | |
|---|---|---|---|---|---|---|---|
| Groups | Vasc* | Infar | Lymph | Throm | Hemo | Fibrin | PMN |
| No presensitization (endpoint) | 3.0 | 3.0 | 1.0 | 4.0 | 3.0 | 3.0 | 3.0 |
| 1". One skin presensitization (endpoint) | 0.0 | 4.0 | 1.0 | 4.0 | 3.0 | 0.0 | 0.0 |
| 2". Cyclosporin A (endpoint) | 0.0 | 4.0 | 0.0 | 3.0 | 3.0 | N/A | N/A |
| 3". BB5.1 (endpoint) | 2.0 | 2.0 | 2.0 | 2.0 | 3.0 | N/A | N/A |
| 4". Cyclophosphamide (endpoint) | 2.0 | 4.0 | 0.0 | 3.0 | 2.0 | N/A | N/A |
| 5". BB5.1 + Cyclosporin A (post-operative day 3) | 0.0 | 1.0 | 1.0 | 0.0 | 0.0 | 0.0 | 0.0 |
| 5". BB5.1 + Cyclosporin A (endpoint) | 2.0 | 2.0 | 2.0 | 2.0 | 2.0 | 0.0 | 0.0 |
| 6". BB5.1 + Cyclophosphamide (endpoint) | 1.0 | 3.0 | 0.0 | 3.0 | 2.0 | N/A | N/A |
| 7". Cyclosporin A + Cyclophosphamide (endpoint) | 0.0 | 1.0 | 1.0 | 2.0 | 3.0 | N/A | N/A |
| 8". BB5.1 + Cyclosporin A + Cyclophosphamide (post-operative day 3) | 0.0 | 0.0 | 1.0 | 0.0 | 0.0 | N/A | N/A |
| 8. BB5.1 + Cyclosporin A + Cyclophosphamide (post-operative day 12) | 0.0 | 0.0 | 1.0 | 0.0 | 0.0 | N/A | N/A |
| 8". BB5.1 + Cyclosporin A + Cyclophosphamide (post-operative day 60) | 0.0 | 0.0 | 1.0 | 0.0 | 0.0 | N/A | N/A |

(continued)

| Median Scores of Histological Changes of Heart Allografts at Necropsy | | | | | | | |
|---|---|---|---|---|---|---|---|
| Groups | Vasc* | Infar | Lymph | Throm | Hemo | Fibrin | PMN |
| 8". BB5.1 + Cyclosporin A + Cyclophosphamide (post-operative day 100) | N/A | N/A | N/A | N/A | N/A | N/A | N/A |
| Median scores: 0 - normal; 1- minimum change; 2 - mild change; 3 - moderate change; 4 - marked change. N/A - not available. *Vasc - vasculitis; Infar - infarction; Lymph - lymphocyte infiltration; Throm - thrombosis; Hemo - hemorrhage; Fibrin - fibrin deposition; PMN - polymorphonuclear cell infiltrate | | | | | | | |

The results shown in Table 5 indicate a difference between the presensitized mouse model and the nonpresensitized mouse model as used in Example 3. The results indicate that in the absence of presensitization, grafts are rejected in approximately 8 days in the absence of treatment with any drugs. Presensitizing the animals causes a more rapid rejection, the rejection of the graft in the presensitized animals being in approximately 3 days in the absence of any drug treatment. Treatment with either BB5.1, cyclosporin A or cyclophosphamide had no effect upon graft survival, with the grafts being rejected in approximately 3-4 days in each of these groups of animals. The combination of BB5.1 and cyclosporin A showed some effect with rejection occurring about day 12. The combination of BB5.1 and cyclophosphamide had no protective effect with rejection occurring about day 3. Similarly the combination of cyclosporin A and cyclophosphamide had essentially no protective effect with rejection occurring at 3-4 days. Very surprisingly, the combination of all three drugs (chronic administration of BB5.1 and cyclosporin plus administration of cyclophosphamide at the time of transplant) showed a highly synergistic effect with all of the mice surviving for more than 100 days. Again, a survival of 100 days in this model is considered to be the gold standard and assumes an indefinite survival.

These results as well as the histological results as shown in Table 6 indicate that the combination of chronic treatment with a complement inhibitor and an immunosuppressant such as cyclosporin A in treating a presensitized mouse results in some attenuation of accelerated rejection. Treatment of these animals additionally with cyclophosphamide at the time of transplant and on the first day after transplant results in a much greater time of survival, no rejection having been seen by at least day 100.

It will be appreciated that the methods and compositions of the instant disclosure can be incorporated in the form of a variety of embodiments, only a few of which are disclosed herein. It will be apparent to the artisan that other embodiments exist and do not depart from the spirit of the disclosure. Thus, the described embodiments are illustrative and should not be construed as restrictive.

LIST OF REFERENCES

The publications and other materials used herein to illuminate the background of the disclosure, and in particular, cases to provide additional details respecting the practice, are incorporated herein by reference in their entirety, and for convenience, are referenced by author and date in the text and respectively grouped in the following List of References.

Abbas AK, et al. (2000). Cellular and Molecular Immunology (4th edition), p. 363-383 (W.B. Saunders Company, New York).

Arp et al. (1996). J. Virol. 70:7349-7359.

Baldwin et al. (2001). Immunity 14:369-376.

Böhmig GA, et al. (2000). Am. J. Kidney Dis. 35:667-673.

Brauer et al. (1995). Transplantation 59:288-293.

Changelian PS, et al. (2003). Science 302:875-878.

Collard et al. (1997). Circulation 96:326-333.

Collins et al. (1999). J. Am. Soc. Nephrol. 10:2208-2214.

Fearon (1983). In Intensive Review of Internal Medicine, 2nd Ed. Fanta and Minaker, eds. Brigham and Women's and Beth Israel Hospitals.

Forbes et al. (1978). Lab. Invest. 39:463-470.

Frei Y, et al. (1987). Mol. Cell. Probes 1:141-149.

Gloor (2005). Contrib. Nephrol. 146:11-21.

Glotz et al. (1993). Transplantation 56:335-337.

Glotz D, et al. (2002). Am. J. Transplant. 2:758-760.

Hakim et al. (1990). Am. J. Kidney Dis. 16:423-431.

Halloran PF, et al. (1992). Transplantation 53:550-555.

Halloran (2003). Am. J. Transplant. 3:639-640.

Haviland DL, et al. (1991). J. Immunol. 146:362-368.

Hiesse C, et al. (1992). Nephrol. Dial. Transplant. 7:944-951.

Hillmen P, et al. (2004). New Engl. J. Med. 350:552-559.

Jeannet M, et al. (1970). New Engl. J Med 282:111-117.

Jones PT;et al. (1986). Nature 321:522-525.

Jose et al. (1983). J. Exp. Med. 158:2177-2182.

Kirschfink (2001). Immunol. Rev. 180:177-189.

Kobayashi et al. (1999). J Biol. Chem. 274:28660-28668.

Kriaa et al. (1995). Nephrol. Dial. Transplant. 10 Suppl. 6:108-110.

Kroshus et al. (1995). Transplantation 60:1194-1202.

Kupiec-Weglinski (1996). Ann. Transplant. 1:34-40.

Kupin et al. (1991). Transplantation 51:324-329.

Liszewski (1993). Fundamental Immunology pp. 917-939.

Mauiyyedi et al. (2002). Curr. Opin. Nephrol. Hypertens. 11:609-618.

Mehra et al. (2003). Curr. Opin. Cardiol. 18:153-158.

Minta JO and Man DP (1977). J. Immunol. 119:1597-1602.

Montgomery RA, et al. (2000). Transplantation 70:887-895.

Olfert et al. (1993) Guide to the care and use of experimental animals (Vol.1). Ottawa: Association of Universities and Colleges of Canada 1.

Opelz G (1992). Transplant. Proc. 24:2342-2355.

OPTN/SRTR Annual Report (2002). Chapter 1 of the Annual Report produced by the Scientific Registry of Transplant Recipients (SRTR) in collaboration with the Organ Procurement and Transplantation Network (OPTN). See http://www.unos.org/data/ar2002/ ar02_chapter_one.htm.

Palmer et al. (1989). Lancet 1:10-12.

Papadimitriou et al. (1991). J. Immunol. 147:212-217.

Park WD et al. (2003). Am. J. Transplant 3:952-960.

Persson NH et al. (1995). Transplant. Proc. 27:3466.

Platt et al. (1999). Mol. Immunol. 36:965-971.

Pratt et al. (1996). Am J Pathol 149:2055-2066.

Pratt et al. (2000). Am. J. Pathol. 157:825-831.

Pruitt et al. (1991). J. Surg. Res. 50:350-355.

Regele H, et al. (2001). Nephrol. Dial. Transplant. 16:2058-2066.

Rocha et al. (2003). Transplantation 75:1490-1495.

Ross et al. (1993). Transplantation 55:785-789.

Saadi et al. (1995). J. Exp. Med 182:1807-1814.

Salama et al. (2001). Am. J. Transplant. 1:260-269.

Schweitzer et al. (2000). Transplantation 70:1531-1536.

Sonnenday et al. (2002). Transplant. Proc. 34:1614-1616.

Stepkowski SM (2000). Exp. Rev. Mol. Med. 21 June, http://www-ermm.cbcu.cam.ac.uk /00001769h.htm.

Taube DH, et al. (1984). Lancet 1:824-828.

Tyan et al. (1994). Transplantation 57:553-562.

Vakeva et al. (1998). Circulation 97:2259-2267.

Vogt W, et al. (1989). Molec. Immunol. 26:1133-1142.

Wang et al. (1995). Proc. Natl. Acad. Sci. U S. A. 92:8955-8959.

Wang et al. (1996). Proc. Natl. Acad. Sci. U. S. A. 93:8563-8568.

Wang et al. (1999). Transplantation 68:1643-1651.

W ang H, et al. (2003). J. Immunol. 171:3823-3836.

Warren et al. (2004). Am. J. Transplant. 4:561-568.

Wetsel RA and Kolb WP (1982). J. Immunol 128:2209-2216.

Wurzner R, et al. (1991). Complement Inflamm. 8:328-340.

Yamamoto KI and Gewurz G (1978). J. Immunol. 120:2008-2015.

PCT Patent Application Publication WO 92/10205

PCT Patent Application Publication WO 00/27421

PCT Patent Application Publication WO 01/37860

U.S. Patent Application Publication US 2003/0129187

U.S. Patent Application Publication US 2003/0180301

U.S. Patent 5,133,916

U.S. Patent 5,573,940

U.S. Patent 5,660,825

U.S. Patent 6,057,131

U.S. Patent 6,100,443

U.S. Patent 6,192,891

U.S. Patent 6,280,957

U.S. Patent 6,302,855

U.S. Patent 6,355,245

U.S. Patent 6,534,058

U.S. Patent 6,821,515

The contents of all references cited herein are incorporated by reference in their entirety.

The invention furthermore comprises the following items:

1. A method to prolong survival of an MHC mismatched allograft in a recipient mammal, said method comprising administering to said mammal a) a drug which inhibits complement activity and b) at least one immunosuppressive drug, wherein said drug which inhibits complement activity is administered chronically.

2. The method of item 1 wherein said mammal is a human.

3. The method of item 2 wherein said MHC mismatched allograft is an HLA mismatched allograft.

4. The method of item 1 wherein said drug which inhibits complement activity inhibits the formation of terminal complement or C5a.

5. The method of item 4 wherein said drug which inhibits formation of terminal complement or C5a is a whole antibody or an antibody fragment.

6. The method of item 5 wherein said whole antibody or antibody fragment is a human, humanized, chimerized or deimmunized antibody or antibody fragment.

7. The method of item 5 wherein said whole antibody or antibody fragment inhibits cleavage of complement C5.

8. The method of item 5 wherein said antibody fragment is selected from the group consisting of an Fab, an F(ab')$_2$, an Fv, a domain antibody, and a single-chain antibody.

9. The method of item 5 wherein said antibody fragment is pexelizumab.

10. The method of item 5 wherein said whole antibody is eculizumab.

11. The method item 10 wherein said eculizumab is administered once every 2 weeks.

12. The method of item 1 wherein said inhibitor of complement activity is selected from the group consisting of a i) soluble complement receptor, ii) CD59, iii) CD55, iv) CD46, and v) an antibody to C5, C6, C7, C8, or C9.

13. The method of item 1 wherein said immunosuppressive drug inhibits T-cell activity or B-cell activity.

14. The method of item 1 wherein said immunosuppressive drug inhibits T-cell activity and B-cell activity.

15. The method of item 1 wherein said immunosuppressive drug is selected from the group consisting of cyclosporin A, tacrolimus, sirolimus, OKT3, a corticosteroid, daclizumab, basiliximab, azathioprene, mycophenolate mofetil, methotrexate, 6-mercaptopurine, anti-T cell antibodies, cyclophosphamide, leflunamide, brequinar, ATG, ALG, 15-deoxyspergualin, and bredinin.

16. The method of item 1 wherein more than one immunosuppressive drug is administered.

17. The method of item 1 wherein said method comprises administering i) a drug which inhibits complement activity and ii) cyclosporin A.

18. The method of item 17 wherein said drug which inhibits complement activity is an antibody which inhibits cleavage of complement C5.

19. The method of item 1 wherein said allograft is selected from the group consisting of i) heart, ii) kidney, iii) lung, iv) pancreas, v) liver, vi) vascular tissue, vii) eye, viii) cornea, ix) lens, x) skin, xi) bone marrow, xii) muscle, xiii) connective tissue, xiv) gastrointestinal tissue, xv) nervous tissue, xvi) bone, xvii) stem cells, xviii) islets, xix) cartilage, xx) hepatocytes, and xxi) hematopoietic cells.

20. The method of item 1 wherein said allograft survives for a time at least 20% longer than would occur if said method were to be performed without said drug which inhibits complement activity.

21. The method of item 1 wherein said allograft survives for a time at least 40% longer than would occur if said method were to be performed without said drug which inhibits complement activity.

22. The method of item 1 wherein said allograft survives for the remaining life-time of said mammal.

23. The method of item 2 wherein said allograft survives for at least six months.

24. The method of item 2 wherein said allograft survives for at least one year.

25. The method of item 2 wherein said allograft survives for at least five years.

26. The method of item 2 wherein said allograft survives for the remaining life-time of said human.

27. The method of item 1 wherein said drug which inhibits complement activity is administered chronically for at least 14 days.

28. The method of item 1 wherein said drug which inhibits complement activity is administered chronically for at least 28 days.

29. The method of item 1 wherein said drug which inhibits complement activity is administered chronically for at least 3 months.

30. The method of item 1 wherein said drug which inhibits complement activity is administered chronically for at least 6 months.

31. The method of item 1 wherein said drug which inhibits complement activity is administered chronically for at least 1 year.

32. The method of item 1 wherein said drug which inhibits complement activity is administered chronically for at least 5 years.

33. The method of item 1 wherein said drug which inhibits complement activity is administered chronically for the remaining life-time of said mammal.

34. The method of item 1 wherein at least one immunosuppressive drug is administered chronically for the remaining life-time of said mammal.

35. The method of item 34 wherein at least cyclosporin A is administered chronically for the remaining life-time of said mammal.

36. A method to prolong survival of an allograft in a recipient mammal presensitized to said allograft, said method comprising administering to said mammal a) a drug which inhibits complement activity and b) at least one immunosuppressive drug, wherein said drug which inhibits complement activity is administered chronically.

37. The method of item 36 wherein said mammal is a human.

38. The method of item 36 wherein said drug which inhibits complement activity inhibits the formation of terminal complement or C5a.

39. The method of item 38 wherein said drug that inhibits formation of terminal complement or C5a is a whole antibody or an antibody fragment.

40. The method of item 39 wherein said whole antibody or antibody fragment is a human, humanized, chimerized or deimmunized antibody or antibody fragment.

41. The method of item 39 wherein said whole antibody or antibody fragment inhibits cleavage of complement C5.

42. The method of item 39 wherein said antibody fragment is selected from the group consisting of an Fab, an $F(ab')_2$, an Fv, a domain antibody, and a single-chain antibody.

43. The method of item 39 wherein said antibody fragment is pexelizumab.

44. The method of item 39 wherein said whole antibody is eculizumab.

45. The method item 44 wherein said eculizumab is administered once every 2 weeks.

46. The method of item 36 wherein said inhibitor of complement activity is selected from the group consisting of a i) soluble complement receptor, ii) CD59, iii) CD55, iv) CD46, and v) an antibody to C5, C6, C7, C8, or C9.

47. The method of item 36 wherein said immunosuppressive drug inhibits T-cell activity or B-cell activity.

48. The method of item 36 wherein said immunosuppressive drug inhibits T-cell activity and B-cell activity.

49. The method of item 36 wherein said immunosuppressive drug is selected from the group consisting of cyclosporin A, tacrolimus, sirolimus, OKT3, a corticosteroid, daclizumab, basiliximab, azathioprene, mycophenolate mofetil, methotrexate, 6-mercaptopurine, anti-T cell antibodies, cyclophosphamide, leflunamide, brequinar, ATG, ALG, 15-deoxyspergualin, and bredinin.

50. The method of item 36 wherein more than one immunosuppressive drug is administered.

51. The method of item 36 wherein said method comprises administering i) a drug which inhibits complement activity and ii) cyclosporin A.

52. The method of item 51 wherein said method comprises administering i) a drug which inhibits complement activity, ii) cyclosporin A, and iii) cyclophosphamide.

53. The method of item 51 wherein said drug which inhibits complement activity is an antibody which inhibits cleavage of complement C5.

54. The method of item 36 wherein said allograft is selected from the group consisting of i) heart, ii) kidney, iii) lung, iv) pancreas, v) liver, vi) vascular tissue, vii) eye, viii) cornea, ix) lens, x) skin, xi) bone marrow, xii) muscle, xiii) connective tissue, xiv) gastrointestinal tissue, xv) nervous tissue, xvi) bone, xvii) stem cells, xviii) islets, xix) cartilage, xx) hepatocytes, and xxi) hematopoietic cells.

55. The method of item 36 wherein said allograft survives for a time at least 20% longer than would occur if said method were to be performed without said drug which inhibits complement activity.

56. The method of item 36 wherein said allograft survives for a time at least 40% longer than would occur if said method were to be performed without said drug which inhibits complement activity.

57. The method of item 36 wherein said allograft survives for the remaining life-time of said mammal.

58. The method of item 37 wherein said allograft survives for at least six months.

59. The method of item 37 wherein said allograft survives for at least one year.

60. The method of item 37 wherein said allograft survives for at least five years.

61. The method of item 37 wherein said allograft survives for the remaining life-time of said human.

62. The method of item 36 wherein said drug which inhibits complement activity is administered chronically for at least 14 days.

63. The method of item 36 wherein said drug which inhibits complement activity is administered chronically for at least 28 days.

64. The method of item 36 wherein said drug which inhibits complement activity is administered chronically for at least 3 months.

65. The method of item 36 wherein said drug which inhibits complement activity is administered chronically for at least 6 months.

66. The method of item 36 wherein said drug which inhibits complement activity is administered chronically for at least 1 year.

67. The method of item 36 wherein said drug which inhibits complement activity is administered chronically for at least 5 years.

68. The method of item 36 wherein said drug which inhibits complement activity is administered chronically for the remaining life-time of said mammal.

69. The method of item 36 wherein at least one immunosuppressive drug is administered chronically for the remaining life-time of said mammal.

70. The method of item 69 wherein at least cyclosporin A is administered chronically for the remaining life-time of said mammal.

71. A method to prolong survival of an allograft in a recipient mammal which is an ABO mismatch to said allograft, said method comprising administering to said mammal a) a drug which inhibits complement activity and b) at least one immunosuppressive drug, wherein said drug which inhibits complement activity is administered chronically.

72. The method of item 71 wherein said mammal is a human.

73. The method of item 71 wherein said drug which inhibits complement activity inhibits the formation of terminal complement or C5a.

74. The method of item 73 wherein said drug which inhibits formation of terminal complement or C5a is a whole antibody or an antibody fragment.

75. The method of item 74 wherein said whole antibody or antibody fragment is a human, humanized, chimerized or deimmunized antibody or antibody fragment.

76. The method of item 74 wherein said whole antibody or antibody fragment inhibits cleavage of complement C5.

77. The method of item 74 wherein said antibody fragment is selected from the group consisting of an Fab, an $F(ab')_2$, an Fv, a domain antibody, and a single-chain antibody.

78. The method of item 74 wherein said antibody fragment is pexelizumab.

79. The method of item 74 wherein said whole antibody is eculizumab.

80. The method item 79 wherein said eculizumab is administered once every 2 weeks.

81. The method of item 71 wherein said inhibitor of complement activity is selected from the group consisting of a i) soluble complement receptor, ii) CD59, iii) CD55, iv) CD46, and v) an antibody to C5, C6, C7, C8, or C9.

82. The method of item 71 wherein said immunosuppressive drug inhibits T-cell activity or B-cell activity.

83. The method of item 71 wherein said immunosuppressive drug inhibits T-cell activity and B-cell activity.

84. The method of item 71 wherein said immunosuppressive drug is selected from the group consisting of cyclosporin A, tacrolimus, sirolimus, OKT3, a corticosteroid, daclizumab, basiliximab, azathioprene, mycophenolate mofetil, methotrexate, 6-mercaptopurine, anti-T cell antibodies, cyclophosphamide, leflunamide, brequinar, ATG, ALG, 15-deoxyspergualin, and bredinin.

85. The method of item 71 wherein more than one immunosuppressive drug is administered.

86. The method of item 71 wherein said method comprises administering i) a drug which inhibits complement activity and ii) cyclosporin A.

87. The method of item 86 wherein said method comprises administering i) a drug which inhibits complement activity, ii) cyclosporin A, and iii) cyclophosphamide.

88. The method of item 86 wherein said drug that inhibits complement activity is an antibody that inhibits cleavage of complement C5.

89. The method of item 71 wherein said allograft is selected from the group consisting of i) heart, ii) kidney, iii) lung, iv) pancreas, v) liver, vi) vascular tissue, vii) eye, viii) cornea, ix) lens, x) skin, xi) bone marrow, xii) muscle, xiii) connective tissue, xiv) gastrointestinal tissue, xv) nervous tissue, xvi) bone, xvii) stem cells, xviii) islets, xix) cartilage, xx) hepatocytes, and xxi) hematopoietic cells.

90. The method of item 71 wherein said allograft survives for a time at least 20% longer than would occur if said method were to be performed without said drug that inhibits complement activity.

91. The method of item 71 wherein said allograft survives for a time at least 40% longer than would occur if said method were to be performed without said drug that inhibits complement activity.

92. The method of item 71 wherein said allograft survives for the remaining life-time of said mammal.

93. The method of item 72 wherein said allograft survives for at least six months.

94. The method of item 72 wherein said allograft survives for at least one year.

95. The method of item 72 wherein said allograft survives for at least five years.

96. The method of item 72 wherein said allograft survives for the remaining life-time of said human.

97. The method of item 71 wherein said drug which inhibits complement activity is administered chronically for at least 14 days.

98. The method of item 71 wherein said drug which inhibits complement activity is administered chronically for at least 28 days.

99. The method of item 71 wherein said drug which inhibits complement activity is administered chronically for at least 3 months.

100. The method of item 71 wherein said drug which inhibits complement activity is administered chronically for at least 6 months.

101. The method of item 71 wherein said drug which inhibits complement activity is administered chronically for at least 1 year.

102. The method of item 71 wherein said drug which inhibits complement activity is administered chronically for at least 5 years.

103. The method of item 71 wherein said drug which inhibits complement activity is administered chronically for the remaining life-time of said mammal.

104. The method of item 71 wherein at least one immunosuppressive drug is administered chronically for the remaining life-time of said mammal.

105. The method of item 104 wherein at least cyclosporin A is administered chronically for the remaining life-time of said mammal.

106. Use of a drug that inhibits complement activity and an immunosuppressive drug in the manufacture of a medicament or medicament package for prolong survival of an allograft in a mammal.

107. The use of item 106, wherein more than one immunosuppressive drug is included in said medicament or medicament package.

108. The use of item 106, wherein said drug that inhibits complement activity and said immunosuppressive drug are in a formulation suitable for concurrent administration to said mammal.

109. The use of item 106, wherein said drug that inhibits complement activity and said immunosuppressive drug are in a formulation or formulations suitable for sequential administration to said mammal.

110. The use of item 106, wherein said drug that inhibits complement activity is in a formulation suitable for chronic administration to said mammal.

111. The use of item 106, wherein said immunosuppressive drug is in a formulation suitable for chronic administration to said mammal.

112. A method to prolong survival of allograft in a recipient mammal, comprising preparing an allograft from a first allograft accommodated by a first recipient mammal, wherein said first recipient mammal, after receiving said first allograft, has been treated with a drug that inhibits complement activity and at least one immunosuppressive agent.

113. An allograft that has prolonged survival in a recipient mammal, wherein said allograft is prepared from a first recipient mammal and was transplanted to the first recipient mammal that has been treated with a drug that inhibits complement activity and at least one immunosuppressive agent.

114. A pharmaceutical package comprising a drug that inhibits complement activity and at least one immunosuppressive agent, wherein said drug and said agent are formulated for chronic administration.

115. The pharmaceutical package of item 114, wherein said drug is an antibody in a lyophilized formulation comprising the antibody and a lyoprotectant.

116. The pharmaceutical package of item 114, wherein said agent is in a lyophilized formulation comprising the agent and a lyoprotectant.

117. The pharmaceutical package of item 114, wherein said drug and said agent are in the same lyophilized formulation comprising said drug, said agent, and a lyoprotectant.

118. The pharmaceutical package of item 114, wherein said drug is in an injection system comprising a syringe that comprises a cartridge, wherein said cartridge contains said drug in a formulation suitable for injection.

119. The pharmaceutical package of item 114, wherein said agent is in an injection system comprising a syringe that comprises a cartridge, wherein said cartridge contains said agent in a formulation suitable for injection.

120. The pharmaceutical package of item 114, wherein said drug and said agent are in an injection system comprising a syringe that comprises a cartridge, wherein said cartridge contains said drug and said agent in a formulation suitable for injection.

121. The pharmaceutical package of item 114, wherein said drug is present in unit dosage form.

122. The pharmaceutical package of item 114, wherein said agent is present in unit dosage form.

123. A lyophilized antibody formulation comprising an antibody that inhibits complement activity and a lyoprotectant, wherein said antibody formulation is suitable for chronic administration.

124. The lyophilized antibody formulation of item 123, wherein the antibody inhibits the formation of terminal complement or C5a.

125. The lyophilized antibody formulation of item 123, wherein the antibody is a whole antibody or an antibody fragment.

126. The lyophilized antibody formulation of item 125, wherein the antibody wherein said whole antibody or antibody fragment is a human, humanized, chimerized or deimmunized antibody or antibody fragment.

127. The lyophilized antibody formulation of item 125, wherein said whole antibody or antibody fragment inhibits cleavage of complement C5.

128. The lyophilized antibody formulation of item 125, wherein said antibody fragment is selected from the group consisting of an Fab, an F(ab')$_2$, an Fv, a domain antibody, and a single-chain antibody.

129. The lyophilized antibody formulation of item 125, wherein said antibody fragment is pexelizumab.

130. The lyophilized antibody formulation of item 125, wherein said whole antibody is eculizumab.

131. An injection system comprising a syringe, wherein said syringe comprises a cartridge containing an antibody that inhibits complement activity and is formulated suitable for injection.

**Claims**

1. A composition for use in prolonging survival of an allograft organ in a human recipient, wherein the composition comprises an inhibitor of human complement component C5, and wherein the inhibitor is to be administered to the human recipient in conjunction with at least one immunosuppressive drug.

2. The composition according to claim 1, wherein the inhibitor inhibits cleavage of C5 into fragments C5a and C5b

3. The composition according to claim 2, wherein the inhibitor is a whole antibody or an antigen-binding fragment thereof that binds to C5.

4. The composition according to claim 3, wherein the whole antibody or antigen-binding fragment thereof is a human, humanized, chimerized, or de-immunized antibody or antigen-binding fragment thereof.

5. The composition according to claim 3, wherein the antigen-binding fragment is selected from the group consisting of an Fab, an F(ab')$_2$, an Fv, and a single chain antibody.

6. The composition according to claim 3, wherein the whole antibody is eculizumab.

7. The composition according to claim 3, wherein the antigen-binding fragment is pexelizumab.

8. The composition according to any one of claims 1 to 7, wherein the allograft is selected from the group consisting of i) heart, ii) kidney, iii) lung, iv) pancreas, v) liver, vi) vascular tissue, vii) eye, viii) cornea, ix) lens, x) skin, xi) bone marrow, xii) muscle, xiii) connective tissue, xiv) gastrointestinal tissue, xv) nervous tissue, xvi) bone, xvii) stem cells, xviii) islets, xix) cartilage, xx) hepatocytes, and xxi) hematopoietic cells.

9. The composition according to any one of claims 1 to 8, wherein the at least one immunosuppressive drug is selected from the group consisting of cyclosporin A, tacrolimus, sirolimus, OKT3, a corticosteroid, daclizumab, basiliximab, azathioprene, mycophenolate mofetil, methotrexate, 6-mercaptopurine, anti-T cell antibodies, cyclophosphamide, leflunamide, brequinar, ATG, ALG, 15-deoxyspergualin, and bredinin.

10. The composition according to any one of claims 1 to 9, wherein the inhibitor is to be used in conjunction with at least two immunosuppressive drugs.

11. The composition according to any one of claims 1 to 10, wherein cyclosporin A is to be administered to the human recipient.

12. The composition according to any one of claims 1 to 11, wherein the human recipient is an MHC mismatch to the allograft, presensitized to the allograft, or an ABO mismatch to the allograft.

13. A composition for use in prolonging survival of an allograft organ in a human recipient, wherein the composition comprises an inhibitor of human complement component C5, and wherein the inhibitor is to be administered to the human recipient in conjunction with at least two immunosuppressive drugs and wherein the human recipient is an MHC mismatch to the allograft, presensitized to the allograft, or an ABO mismatch to the allograft.

A

B

**Figure 1A, B**

**Figure 1C, D**

**A**

Figure 2A

B

Figure 2B

Figure 3

**Figure 4A, B**

C

D

Figure 4C, D

Figure 5A, B

**Figure 6**

Figure 7

**A**

**B**

Figure 8

Figure 9

Europäisches
Patentamt

European
Patent Office

Office européen
des brevets

# EUROPEAN SEARCH REPORT

Application Number

EP 14 17 7646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | KROSHUS TIMOTHY J ET AL: "Complement inhibition with an anti-C5 monoclonal antibody prevents acute cardiac tissue injury in an ex vivo model of pig-to-human xenotransplantation", TRANSPLANTATION (BALTIMORE), vol. 60, no. 11, 1995, pages 1194-1202, XP002356728, ISSN: 0041-1337 * the whole document * | 1-13 | INV. A61K39/395 C07K16/18 |
| A,D | WANG H ET AL: "COMPLEMENT INHIBITION WITH AN ANTI-C5 MONOCLONAL ANTIBODY PREVENTS HYPERACUTE REJECTION IN A XENOGRAFT HEART TRANSPLANTATION MODEL", TRANSPLANTATION, WILLIAMS AND WILKINS, BALTIMORE, MD, US, vol. 68, no. 11, 15 December 1999 (1999-12-15), pages 1643-1651, XP001095658, ISSN: 0041-1337 * the whole document * | 1-13 | |
| A,D | US 6 355 245 B1 (EVANS MARK J ET AL) 12 March 2002 (2002-03-12) * the whole document * | 1-13 | TECHNICAL FIELDS SEARCHED (IPC) C07K |
| A | US 2002/041875 A1 (FUNG MICHAEL S.C ET AL) 11 April 2002 (2002-04-11) * the whole document * | 1-13 | |

-/--

The present search report has been drawn up for all claims

| Place of search | Date of completion of the search | Examiner |
|---|---|---|
| Munich | 4 November 2014 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 14 17 7646

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | VAKEVA ANTTI P ET AL: "Myocardial infarction and apoptosis after myocardial ischemia and reperfusion: Role of the terminal complement components and inhibition by anti-C5 therapy", CIRCULATION, vol. 97, no. 22, 9 June 1998 (1998-06-09), pages 2259-2267, XP002356729, ISSN: 0009-7322 * the whole document * | 1-13 | |
| T | WANG HAO ET AL: "Prevention of acute vascular rejection by a functionally blocking anti-C5 monoclonal antibody combined with cyclosporine", TRANSPLANTATION (HAGERSTOWN), vol. 79, no. 9, May 2005 (2005-05), pages 1121-1127, XP002356730, ISSN: 0041-1337 * the whole document * | 1-13 | |
| | | | TECHNICAL FIELDS SEARCHED (IPC) |

The present search report has been drawn up for all claims

| Place of search | Date of completion of the search | Examiner |
|---|---|---|
| Munich | 4 November 2014 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 17 7646

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2014

| Patent document cited in search report | | Publication date | Patent family member(s) | | Publication date |
|---|---|---|---|---|---|
| US 6355245 | B1 | 12-03-2002 | AT | 448791 T | 15-12-2009 |
| | | | AU | 2474795 A | 29-11-1995 |
| | | | BR | 9507594 A | 16-09-1997 |
| | | | CA | 2189015 A1 | 09-11-1995 |
| | | | CA | 2690298 A1 | 09-11-1995 |
| | | | DE | 122009000075 I1 | 12-05-2010 |
| | | | DK | 0758904 T3 | 18-01-2010 |
| | | | EP | 0758904 A1 | 26-02-1997 |
| | | | EP | 2112165 A2 | 28-10-2009 |
| | | | EP | 2270046 A2 | 05-01-2011 |
| | | | EP | 2270047 A2 | 05-01-2011 |
| | | | EP | 2298808 A1 | 23-03-2011 |
| | | | ES | 2336051 T3 | 07-04-2010 |
| | | | JP | 3734266 B2 | 11-01-2006 |
| | | | JP | 4294596 B2 | 15-07-2009 |
| | | | JP | 5047996 B2 | 10-10-2012 |
| | | | JP | H10500289 A | 13-01-1998 |
| | | | JP | 2005185286 A | 14-07-2005 |
| | | | JP | 2006020633 A | 26-01-2006 |
| | | | JP | 2009165471 A | 30-07-2009 |
| | | | JP | 2012095650 A | 24-05-2012 |
| | | | KR | 100381128 B1 | 29-09-2003 |
| | | | KR | 20040000388 A | 03-01-2004 |
| | | | NL | 300433 I1 | 01-03-2010 |
| | | | PT | 758904 E | 10-02-2010 |
| | | | US | 6074642 A | 13-06-2000 |
| | | | US | 6355245 B1 | 12-03-2002 |
| | | | WO | 9529697 A1 | 09-11-1995 |
| US 2002041875 | A1 | 11-04-2002 | AT | 449791 T | 15-12-2009 |
| | | | AU | 9659401 A | 22-04-2002 |
| | | | CA | 2424379 A1 | 18-04-2002 |
| | | | DK | 1325033 T3 | 06-04-2010 |
| | | | EP | 1325033 A2 | 09-07-2003 |
| | | | EP | 2113516 A1 | 04-11-2009 |
| | | | EP | 2281840 A2 | 09-02-2011 |
| | | | ES | 2333775 T3 | 01-03-2010 |
| | | | ES | 2483991 T3 | 08-08-2014 |
| | | | PT | 1325033 E | 15-04-2010 |
| | | | US | 2002041875 A1 | 11-04-2002 |
| | | | WO | 0230985 A2 | 18-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 57144404 P
- US 6280957 B
- WO 0137860 A
- WO 0027421 A
- US 20030180301 A
- US 6355245 B
- WO 9210205 A
- US 6057131 A
- US 5135916 A, Sims
- US 5573940 A, Sims
- US 6100443 A
- US 6534058 B
- US 20030129187 A
- US 5660825 A
- US 6302855 B
- US 6821515 B
- US 5308341 A
- US 6192891 B
- US 5133916 A

### Non-patent literature cited in the description

- Peptide and Protein Drug Delivery. Marcel Dekker, Inc, 1991, 247-301
- JONES, A. *Adv. Drug Delivery Rev.,* 1993, vol. 10, 29-90
- ABBAS AK et al. Cellular and Molecular Immunology. W.B. Saunders Company, 2000, 363-383
- ARP et al. *J. Virol.,* 1996, vol. 70, 7349-7359
- BALDWIN et al. *Immunity,* 2001, vol. 14, 369-376
- BÖHMIG GA et al. *Am. J. Kidney Dis.,* 2000, vol. 35, 667-673
- BRAUER et al. *Transplantation,* 1995, vol. 59, 288-293
- CHANGELIAN PS et al. *Science,* 2003, vol. 302, 875-878
- COLLARD et al. *Circulation,* 1997, vol. 96, 326-333
- COLLINS et al. *J. Am. Soc. Nephrol.,* 1999, vol. 10, 2208-2214
- FEARON. Intensive Review of Internal Medicine. 1983
- FORBES et al. *Lab. Invest.,* 1978, vol. 39, 463-470
- FREI Y et al. *Mol. Cell. Probes,* 1987, vol. 1, 141-149
- GLOOR. *Contrib. Nephrol.,* 2005, vol. 146, 11-21
- GLOTZ et al. *Transplantation,* 1993, vol. 56, 335-337
- GLOTZ D et al. *Am. J. Transplant.,* 2002, vol. 2, 758-760
- HAKIM et al. *Am. J. Kidney Dis.,* 1990, vol. 16, 423-431
- HALLORAN PF et al. *Transplantation,* 1992, vol. 53, 550-555
- HALLORAN. *Am. J. Transplant.,* 2003, vol. 3, 639-640
- HAVILAND DL et al. *J. Immunol.,* 1991, vol. 146, 362-368
- HIESSE C et al. *Nephrol. Dial. Transplant.,* 1992, vol. 7, 944-951
- HILLMEN P et al. *New Engl. J. Med.,* 2004, vol. 350, 552-559
- JEANNET M et al. *New Engl. J Med,* 1970, vol. 282, 111-117
- JONES PT. *Nature,* 1986, vol. 321, 522-525
- JOSE et al. *J. Exp. Med.,* 1983, vol. 158, 2177-2182
- KIRSCHFINK. *Immunol. Rev.,* 2001, vol. 180, 177-189
- KOBAYASHI et al. *J Biol. Chem.,* 1999, vol. 274, 28660-28668
- KRIAA et al. *Nephrol. Dial. Transplant.,* 1995, vol. 10 (6), 108-110
- KROSHUS et al. *Transplantation,* 1995, vol. 60, 1194-1202
- KUPIEC-WEGLINSKI. *Ann. Transplant.,* 1996, vol. 1, 34-40
- KUPIN et al. *Transplantation,* 1991, vol. 51, 324-329
- LISZEWSKI. *Fundamental Immunology,* 1993, 917-939
- MAUIYYEDI et al. *Curr. Opin. Nephrol. Hypertens.,* 2002, vol. 11, 609-618
- MEHRA et al. *Curr. Opin. Cardiol.,* 2003, vol. 18, 153-158
- MINTA JO ; MAN DP. *J. Immunol.,* 1977, vol. 119, 1597-1602
- MONTGOMERY RA et al. *Transplantation,* 2000, vol. 70, 887-895

- **OLFERT et al.** Guide to the care and use of experimental animals. Association of Universities and Colleges of Canada, 1993, vol. 1, 1
- **OPELZ G.** *Transplant. Proc.,* 1992, vol. 24, 2342-2355
- Annual Report produced by the Scientific Registry of Transplant Recipients (SRTR) in collaboration with the Organ Procurement and Transplantation Network (OPTN). OPTN/SRTR Annual Report. 2002
- **PALMER et al.** *Lancet,* 1989, vol. 1, 10-12
- **PAPADIMITRIOU et al.** *J. Immunol.,* 1991, vol. 147, 212-217
- **PARK WD et al.** *Am. J. Transplant,* 2003, vol. 3, 952-960
- **PERSSON NH et al.** *Transplant. Proc.,* 1995, vol. 27, 3466
- **PLATT et al.** *Mol. Immunol.,* 1999, vol. 36, 965-971
- **PRATT et al.** *Am J Pathol,* 1996, vol. 149, 2055-2066
- **PRATT et al.** *Am. J. Pathol.,* 2000, vol. 157, 825-831
- **PRUITT et al.** *J. Surg. Res.,* 1991, vol. 50, 350-355
- **REGELE H et al.** *Nephrol. Dial. Transplant.,* 2001, vol. 16, 2058-2066
- **ROCHA et al.** *Transplantation,* 2003, vol. 75, 1490-1495
- **ROSS et al.** *Transplantation,* 1993, vol. 55, 785-789
- **SAADI et al.** *J. Exp. Med,* 1995, vol. 182, 1807-1814
- **SALAMA et al.** *Am. J. Transplant.,* 2001, vol. 1, 260-269
- **SCHWEITZER et al.** *Transplantation,* 2000, vol. 70, 1531-1536
- **SONNENDAY et al.** *Transplant. Proc.,* 2002, vol. 34, 1614-1616
- **STEPKOWSKI SM.** *Exp. Rev. Mol. Med.,* 21 June 2000, http://www-ermm.cbcu.cam.ac.uk /00001769h.htm
- **TAUBE DH et al.** *Lancet,* 1984, vol. 1, 824-828
- **TYAN et al.** *Transplantation,* 1994, vol. 57, 553-562
- **VAKEVA et al.** *Circulation,* 1998, vol. 97, 2259-2267
- **VOGT W et al.** *Molec. Immunol.,* 1989, vol. 26, 1133-1142
- **WANG et al.** *Proc. Natl. Acad. Sci. U S A.,* 1995, vol. 92, 8955-8959
- **WANG et al.** *Proc. Natl. Acad. Sci. U. S. A.,* 1996, vol. 93, 8563-8568
- **WANG et al.** *Transplantation,* 1999, vol. 68, 1643-1651
- **W ANG H et al.** *J. Immunol.,* 2003, vol. 171, 3823-3836
- **WARREN et al.** *Am. J. Transplant.,* 2004, vol. 4, 561-568
- **WETSEL RA ; KOLB WP.** *J. Immunol,* 1982, vol. 128, 2209-2216
- **WURZNER R et al.** *Complement Inflamm.,* 1991, vol. 8, 328-340
- **YAMAMOTO KI ; GEWURZ G.** *J. Immunol.,* vol. 120, 2008-2015